# EUROPEAN PATENT APPLICATION

(11) **EP 4 792 888 A1**
(43) Date of publication of application: **19.08.2026**
(21) Application number: 25158102.1
(22) Date of filing: 14.02.2025
(51) Int. Cl.: A61P 31/04, C07K 7/06, A61K 38/00

(54) **NOVEL DAROBACTIN DERIVATIVES CHARACTERIZED BY HIGH ACTIVITY AGAINST GRAM-NEGATIVE PATHOGENS**

(71) Applicant: Justus-Liebig-Universität Gießen, 35390 Gießen (DE)
(72) Inventor: Schäberle, Till, 35394 Gießen (DE); Böhringer, Nils, 35390 Gießen (DE); Wuisan, Zerlina G, 35392 Gießen (DE); Marner, Michael, 60486 Frankfurt am Main (DE)
(74) Representative: Vossius & Partner Patentanwälte Rechtsanwälte mbB

(57) **Abstract**

The present invention relates to novel darobactin derivatives of formula (I): 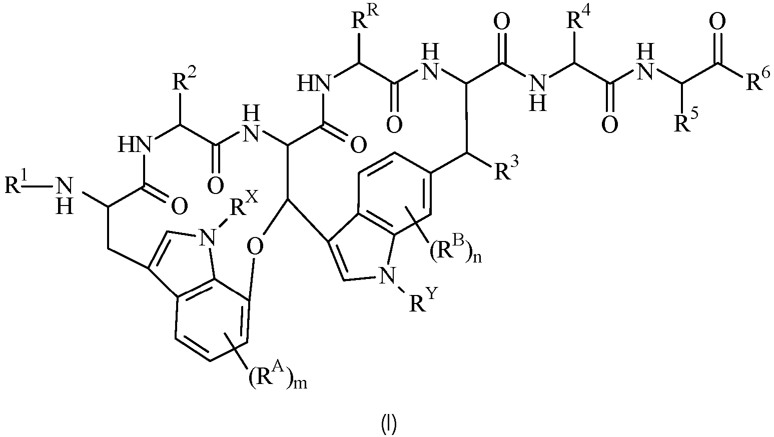 and their pharmaceutically acceptable salts and their use in therapy. Particularly preferred compounds of formula (I) are modified heptapeptides with an amino acid sequence W1-N2-W3-R4-K5-R6-F7 (SEQ ID NO.: 1) and W1-N2-W3-R4-K5-T6-F7 (SEQ ID NO.: 2) (as well as their W7 variants). Instant compounds are particularly useful as active pharmaceutical ingredients (APIs) in medicaments against infectious diseases caused by Gram-negative bacteria.

## Description

The present invention relates to novel darobactin derivatives of formula (I), and their pharmaceutically acceptable salts and their use in therapy. Particularly preferred compounds of formula (I) are modified heptapeptides with an amino acid sequence W1-N2-W3-R4-K5-R6-F7 (SEQ ID NO.: 1) and W1-N2-W3-R4-K5-T6-F7 (SEQ ID NO.: 2) (as well as their W7 variants). Instant compounds are particularly useful as active pharmaceutical ingredients (APIs) in medicaments against infectious diseases caused by Gram-negative bacteria.

The emerging number of antibiotic-resistant bacterial pathogens leads to increasing mortality rates in humans. The current need for novel antibiotics is especially acute for drug-resistant Gram- negative pathogens and the World Health Organization recommended focusing on the discovery and development of new antibiotics with anti-Gram-negative activity. These microorganisms have a highly restrictive permeability barrier, which limits penetration of most compounds. As a result, the last class of antibiotics acting against Gram-negative bacteria was developed in the 60s years of the previous century. It is difficult to find compounds acting against Gram-negative bacteria. Gram-negative bacteria have an outer membrane to protect themselves from unwanted compounds. This membrane is decorated with an outer layer of negatively charged lipopolysaccharide (LPS), which serves as a barrier for large and hydrophobic compounds. The inner membrane limits the permeability by hydrophilic compounds.

As a result, the combined barrier (outer membrane plus inner membrane) restricts all molecules, and nutrients enter through outer membrane porins and specialized transporters. Drugs that leak through the barrier are extruded by trans-envelope multidrug resistance pumps (MDRs) that recognize amphiphilic compounds, which most drugs are in order to pass hydrophilic regions of cells as well as hydrophobic regions (e. g. membranes). Thus, there is great need within the state of the art for APIs with different working-mechanisms, being unhindered by the triple-defenseline of Gram-negative bacteria (outer membrane, inner membrane and MDRs).

Darobactins (DARs) are bicyclic heptapeptides have previously been reported as promising agents with high activity against Gram-negative pathogens. This includes darobactin A (DAR-A), darobactin B (DAR-B), darobactin B9 (DAR B9) and others (WO2020018173A1, WO2021098989A1, WO2022175443A1). Darobactin and its derivatives are ribosomally synthesized and post-translationally modified peptide (RiPP) antibiotics, which were initially identified from bacteria belonging to the genus *Photorhabdus.* In addition, the corresponding biosynthetic gene cluster (BGC) was identified and subsequently detected in several bacterial genera. DARs target the outer membrane protein BamA and are therefore specific for Gram-negative bacteria. This, together with the convincing in vivo activities in mouse infection models, have made them promising candidates for further research.

Novel active compounds from this class, in particular those showing improved activity against some or all relevant Gram-negative bacteria, continue to be desirable in this challenging environment of rising antimicrobial resistance. Thus, it was an objective technical problem of the present invention to provide improved substances that can be used against Gram-negative bacteria. Particularly desired are novel compounds that can overcome already existing resistance of Gram-negative bacteria to presently used drugs.

In the context of the present invention, novel darobactin derivatives based on the cyclic heptapeptide framework and bearing an arginine residue or its derivative at position 4, have been prepared. The so modified darobactin derivatives have unexpectedly shown very promising activity against Gram-negative pathogenic bacteria. In particular the exemplified darobactin R (DAR-R), a modified heptapeptide with an amino acid sequence W1-N2-W3-R4-K5-R6-F7 (SEQ ID NO.: 1), has been shown to be orders of magnitude more potent against the strains of *E. coli, P. aeruginosa, K. pneumoniae,* and others strains, as shown in Table 1, when compared to other established darobactins, such as darobactin A, darobactin B and darobactin B9. Furthermore, darobactin R has been shown to be also active against a number of multi-drug resistant strains of E. *coli* and *P. aeruginosa* (see Table 2). Furthermore, improved activity of new darobactin derivatives against *E. coli, K. pneumoniae, P. aeruginosa* and A. *baumannii* has further been shown for said darobactin R, as well as darobactin R1 (DAR-R1), a modified heptapeptide with an amino acid sequence W1-N2-W3-R4-K5-T6-F7 (SEQ ID NO.: 2) (see Table 6). It has been further shown that, in addition to the presence of arginine or its derivative at position 4, particularly desirable is further presence of an arginine or its derivative at position 6, as demonstrated in the case of newly prepared darobactin R.

Accordingly, in a first embodiment, the present invention relates to a compound of formula (I): 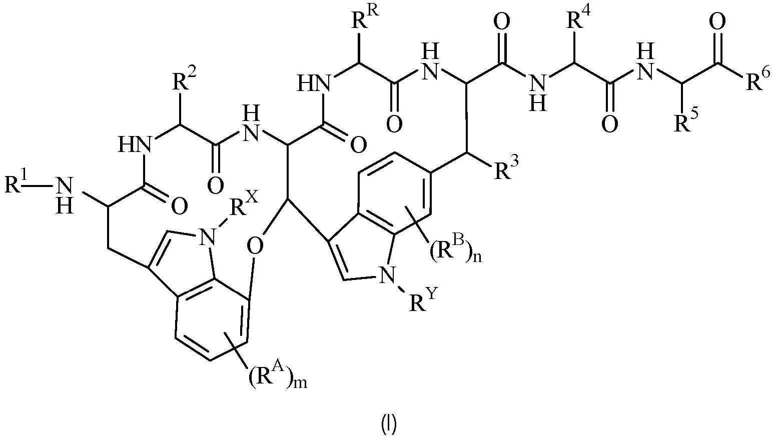 or a pharmaceutically acceptable salt thereof, wherein:
R¹ is selected from hydrogen, C₁₋₅ alkyl, C₂₋₅ alkenyl, C₂₋₅ alkynyl, C₁₋₅ haloalkyl, -(C₀₋₅ alkylene)-CO-(C₁₋₅ alkyl), -(C₀₋₅ alkylene)-CO-carbocyclyl, -(C₀₋₅ alkylene)-CO-heterocyclyl, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-CO-carbocyclyl, in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-CO-heterocyclyl, in said -(C₀₋₅ alkylene)-SO₂-heterocyclyl or in said -(C₀₋₅ alkylene)-SO₂-NH-heterocyclyl are each optionally substituted with one or more groups R^{S};
R² is selected from hydrogen, C₁₋₅ alkyl, C₂₋₅ alkenyl, C₂₋₅ alkynyl, -(C₁₋₅ alkylene)-OH, -(C₁₋₅ alkylene)-SH, -(C₁₋₅ alkylene)-S(C₁₋₅ alkyl), -(C₁₋₅ alkylene)-NH₂, -(C₁₋₅ alkylene)-NH-C(=NH)-NH₂, -(C₁₋₅ alkylene)-COOH, -(C₁₋₅ alkylene)-CO-NH₂, phenyl, -(C₁₋₅ alkylene)-phenyl, cycloalkyl, -(C₁₋₅ alkylene)-cycloalkyl, heteroaryl, -(C₁₋₅ alkylene)-heteroaryl, heterocycloalkyl, and -(C₁₋₅ alkylene)-heterocycloalkyl, wherein said phenyl, the phenyl moiety in said -(C₁₋₅ alkylene)-phenyl, said cycloalkyl, the cycloalkyl moiety in said -(C₁₋₅ alkylene)-cycloalkyl, said heteroaryl, the heteroaryl moiety in said -(C₁₋₅ alkylene)-heteroaryl, said heterocycloalkyl, and the heterocycloalkyl moiety in said -(C₁₋₅ alkylene)-heterocycloalkyl, are each optionally substituted with one or more groups R^{S};
R³ is selected from hydrogen, -(C₁₋₅ alkylene)-NH-R^{N}, and -(C₁₋₅ alkylene)-NH-C(=NH)-NH₂, wherein R^{N} is selected from hydrogen, C₁₋₅ alkyl, C₂₋₅ alkenyl, C₂₋₅ alkynyl, C₁₋₅ haloalkyl, -(C₀₋₅ alkylene)-CO-(C₁₋₅ alkyl), -(C₀₋₅ alkylene)-CO-carbocyclyl, -(C₀₋₅ alkylene)-CO-heterocyclyl, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-CO-carbocyclyl, in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-CO-heterocyclyl, in said -(C₀₋₅ alkylene)-SO₂-heterocyclyl or in said -(C₀₋₅ alkylene)-SO₂-NH-heterocyclyl, are each optionally substituted with one or more groups R^{S};
R⁴ is selected from hydrogen, -C₁₋₅ alkyl, -(C₁₋₅ alkylene)-NH-C(=NH)-NH₂, -(C₁₋₅ alkylene)-CO-NH₂, -(C₁₋₅ alkylene)-(OH), and -(C₁₋₅ alkylene)-heterocyclyl, wherein the heterocyclyl moiety in said -(C₁₋₅ alkylene)-heterocyclyl is optionally substituted with one or more groups R^{S};
R⁵ is selected from hydrogen, C₁₋₅ alkyl, C₂₋₅ alkenyl, C₂₋₅ alkynyl, -(C₁₋₅ alkylene)-OH, -(C₁₋₅ alkylene)-SH, phenyl, -(C₁₋₅ alkylene)-phenyl, cycloalkyl, -(C₁₋₅ alkylene)-cycloalkyl, heteroaryl, -(C₁₋₅ alkylene)-heteroaryl, heterocycloalkyl, and -(C₁₋₅ alkylene)-heterocycloalkyl, wherein said phenyl, the phenyl moiety in said -(C₁₋₅ alkylene)-phenyl, said cycloalkyl, the cycloalkyl moiety in said -(C₁₋₅ alkylene)-cycloalkyl, said heteroaryl, the heteroaryl moiety in said -(C₁₋₅ alkylene)-heteroaryl, said heterocycloalkyl, and the heterocycloalkyl moiety in said -(C₁₋₅ alkylene)-heterocycloalkyl, are each optionally substituted with one or more groups R^{S};
R⁶ is selected from -OH, -NH₂, -O(C₁₋₅ alkyl), -NH(C₁₋₅ alkyl) and N(C₁₋₅ alkyl)(C₁₋₅ alkyl);
R^{R} is -(C₁₋₅ alkylene)-NH-C(=NH)-NH₂;
R^{A} and R^{B} are each individually selected from halogen, and C₁₋₅ alkyl;
R^{X} and R^{Y} are each individually selected from hydrogen, C₁₋₅ alkyl, C₁₋₅ haloalkyl, and cycloalkyl;
each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NO₂, -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -(C₀₋₃ alkylene)-carbocyclyl, and -(C₀₋₃ alkylene)-heterocyclyl, wherein the carbocyclyl moiety in said -(C₀₋₃ alkylene)-carbocyclyl and the heterocyclyl moiety in said -(C₀₋₃ alkylene)-heterocyclyl are each optionally substituted with one or more groups independently selected from C₁₋₄ alkyl, halogen, -CN, -NO₂, -OH, -O-(C₁₋₄ alkyl), -SH, -S-(C₁₋₄ alkyl), -NH₂, -NH(C₁₋₄ alkyl), -N(C₁₋₄ alkyl)(C₁₋₄ alkyl), -COOH, -COO(C₁₋₄ alkyl), -CONH₂, -CONH(C₁₋₄ alkyl), -CON(C₁₋₄ alkyl)(C₁₋₄ alkyl), -NHCO(C₁₋₄ alkyl) and -N(C₁₋₄ alkyl)-CO(C₁₋₄ alkyl);
m is 0 or 1; and
n is 0 or 1.

The present invention also relates, in one embodiment, to a pharmaceutical composition comprising a compound of formula (I) or a pharmaceutically acceptable salt thereof, and a pharmaceutically acceptable excipient. Accordingly, the invention relates to a compound of formula (I) or a pharmaceutically acceptable salt thereof, or a pharmaceutical composition comprising any of the aforementioned entities and a pharmaceutically acceptable excipient, for use as a medicament.

The invention further relates in one embodiment to a compound of formula (I) or a pharmaceutically acceptable salt thereof, or a pharmaceutical composition comprising any of the aforementioned entities and a pharmaceutically acceptable excipient, for use in the treatment or prevention of a bacterial infectious disease. It is preferred that said bacterial infectious diseases is caused by a Gram-negative bacterium.

Moreover, in one embodiment the present invention relates to use of a compound of formula (I) or a pharmaceutically acceptable salt thereof in the preparation of a medicament for the treatment or prevention of a bacterial infectious disease. It is preferred that said bacterial infectious diseases is caused by a Gram-negative bacterium.

The invention likewise relates to a method of treating or preventing a bacterial infectious disease, the method comprising administering a compound of formula (I) or a pharmaceutically acceptable salt thereof, or of a pharmaceutical composition comprising any of the aforementioned entities and a pharmaceutically acceptable excipient, to a subject (preferably a human) in need thereof. It is to be understood that a therapeutically effective amount of said compound or said pharmaceutical composition is to be administered. It is further preferred that said bacterial infectious diseases is caused by a Gram-negative bacterium.

The present invention is illustrated by the appended figures, which serve merely an illustrative purpose and are not meant to be construed as limiting the scope of the invention in any way.

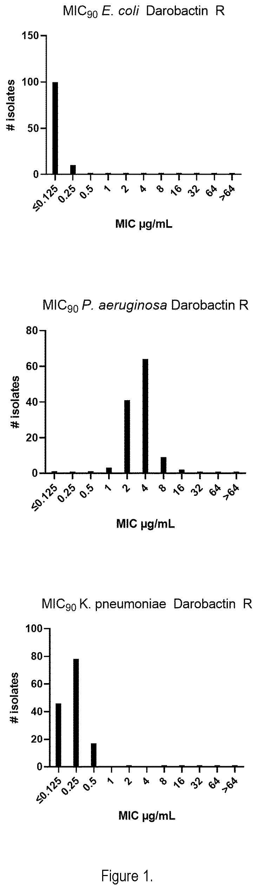

- **Fig. 1:**: Superior minimum inhibitory concentration (MIC₉₀) exhibited by darobactin R, at which ≥90% of the strains of *E. coli, P. aeruginosa* and *K. pneumoniae* are inhibited. Activity of darobactin R was determined against a cohort of *E. coli* (n=111), *K. pneumoniae* (n=143) and *P. aeruginosa* (n=121) isolates. The activity of darobactin R is 4-fold stronger against all three species compared to reference darobactin B.

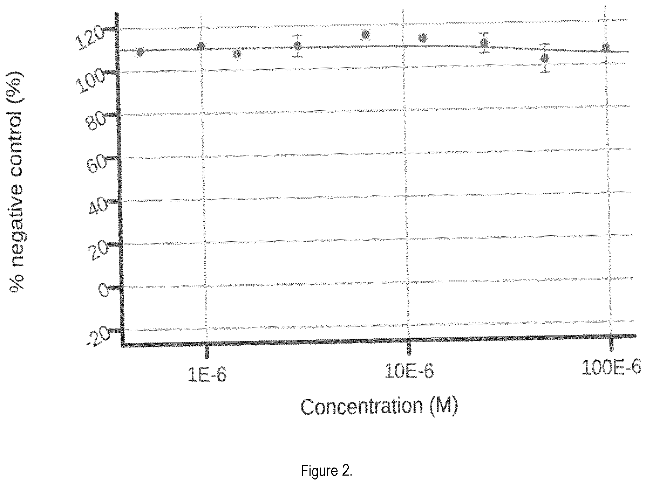

- **Fig. 2:**: Excellent in *vitro* toxicity profile of darobactin R (in a 1:2 dilution series; 9 points ranging from 100-0.5 µM) was shown in a study with HepG2 cells. The assay plates were incubated for 72 hours, before the cell viability of determined. Results are expressed relative to a negative (maximal cell viability) control. No precipitation or cell toxicity was observed at the tested concentrations.

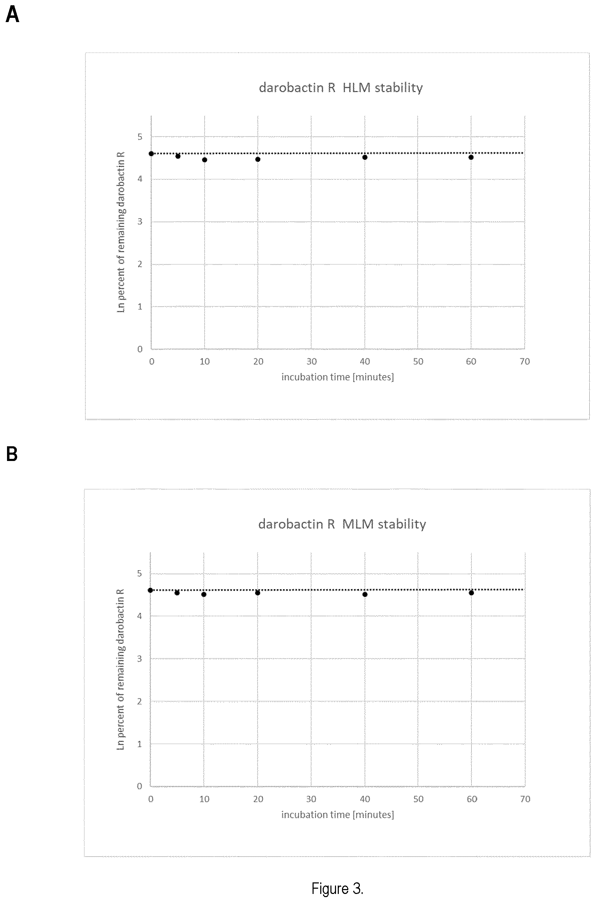

- **Fig. 3:**: Excellent metabolic stability displayed by darobactin R in both **(A)** human liver microsomes (HLM) and **(B)** mouse liver microsomes (MLM) at a compound concentration of 1 µM in 100 mM KPO4 buffer pH 7.4 in a total incubation volume of 500 µL.

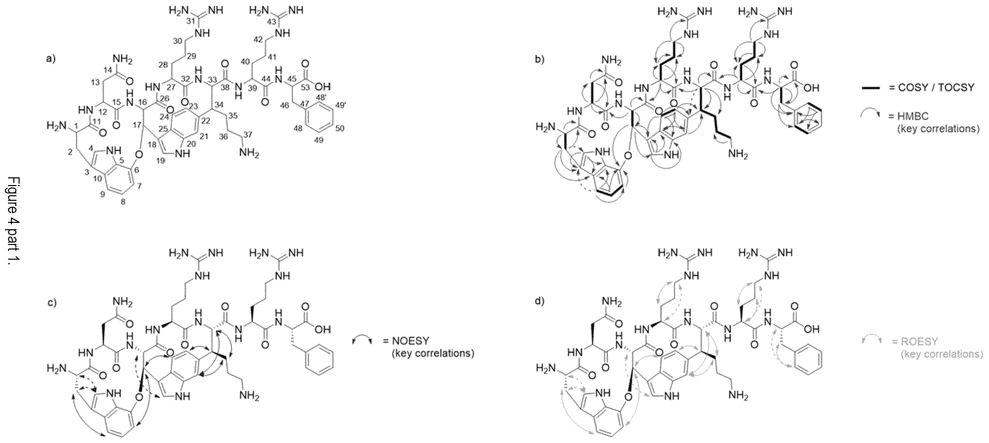

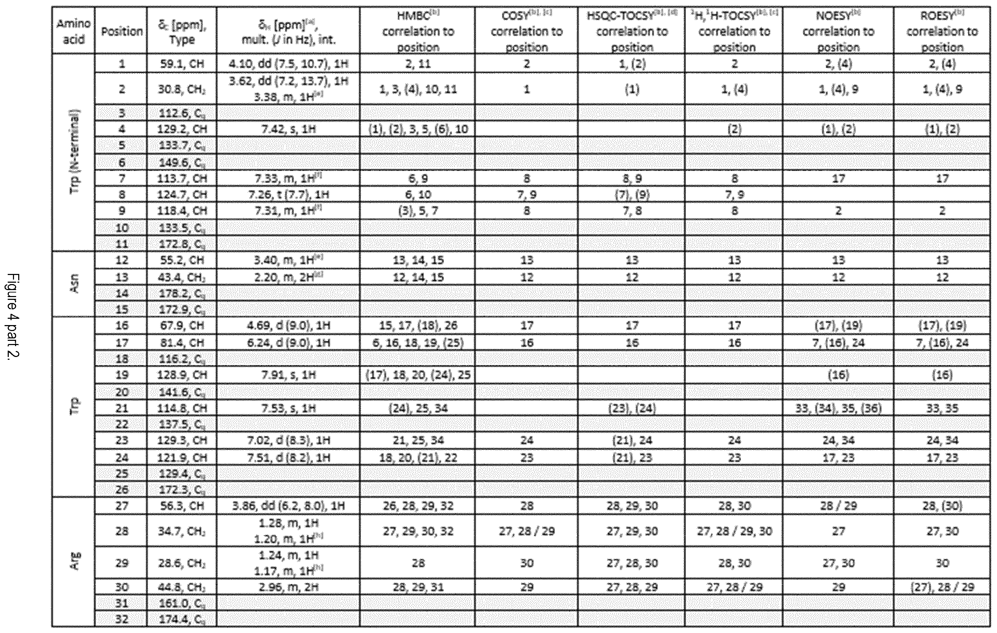

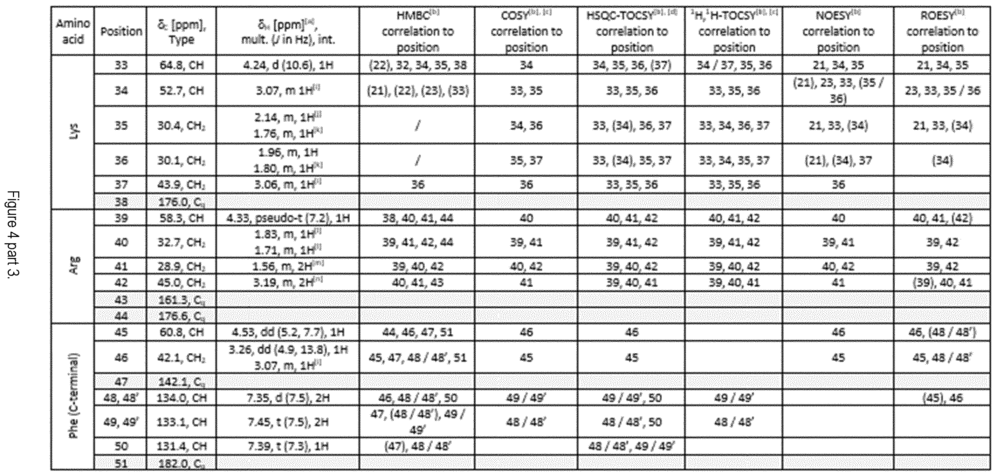

- **Fig. 4:**: NMR-based confirmation of the structure of darobactin R. Part 1: (a) Structure of darobactin R, including the atom numbering used for NMR structure elucidation. (b) COSY, TOCSY, and key HMBC correlations of darobactin R. (c) Key NOESY correlations of darobactin R. (d) Key ROESY correlations of darobactin R (Dashed arrows indicate weak correlation signals). Parts 2 and 3: ¹H (700 MHz) and ¹³C (176.1 MHz) NMR data of darobactin R (D₂O; δ in ppm) alongside correlation data from HMBC, COSY, TOCSY, and NOESY experiments. For ¹³C measurements 3-(trimethylsilyl)propionic-2,2,3,3-d₄ acid sodium salt (TSPA) was used as external standard. The following abbreviations are used in this table: mult.: multiplicity, int.: integral, obs.: obscured. [a] The ¹H shifts of multiplets were extracted from the HSQC spectrum (700 MHz, 175 MHz). [b] Brackets indicate weak correlation signals. [c] Geminal coupling (²*J*_{HH}) is not displayed. [d] ¹J_{CH} coupling is not displayed. [e] The observed integral for this signal was 2H due to overlap of the proton signals for H-2b and H-12. Thus, for each of these positions an integral of 1H was assigned. [f] The multiplicity of H-7 and H-9 is denoted as m because the true multiplicity is not resolved due to overlap of both signals. The signal for H-7 and H-9 is observed as a pseudo-triplet having an integral of 2H. Therefore, for each of these positions an integral of 1H was assigned. [g] This peak is partially overlapped by an impurity. Still, the observed integral is 2H. [h] The observed integral for this signal was 2H due to overlap of the proton signals for H-28b and H-29b. Thus, for each of these positions an integral of 1H was assigned. [i] The observed integral for this signal was 5H due to overlap of the proton signals for H-34, H-37, and H-46b with an impurity. Therefore, for H-34, and H-46b an integral of 1H, each, was assigned, while for H-37 an integral of 2H was assigned. [j] The observed integral for this signal was 2H due to overlap of the proton signal for H-35a with an impurity. The expected integral for H-35a is 1H. [k] The observed integral for this signal was 2H due to overlap of the proton signals for H-35b and H-36b. Thus, for each of these positions an integral of 1H was assigned. Furthermore, partial overlap with the proton signals for H-40a and H-40b can be observed. [I] For both signals, H-40a and H-40b, the observed integrals were 2H each. This was attributed to partial overlap with the proton signals for H-35b and H-36b as well as to overlap with a residual impurity. The expected integrals for H-40a and H-40b are 1H each. [m] The observed integral for this signal was 3H due to overlap of the proton signal for H-41 with an impurity. The expected integral for H-41 is 2H. [n] The observed integral for this signal was 3H due to overlap of the proton signal for H-42 with an impurity. The expected integral for H-42 is 2H.

As explained above, the present invention relates to a compound of formula (I): 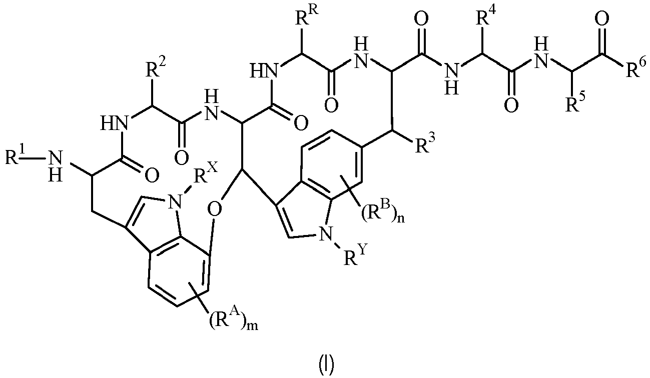 or a pharmaceutically acceptable salt thereof.

The following detailed description of the compound of formula (I) relates to all embodiments of the present invention and is also applicable to all salts (in particular pharmaceutically acceptable salts) of the compound of formula (I).

In formula (I), R' is selected from hydrogen, C₁₋₅ alkyl, C₂₋₅ alkenyl, C₂₋₅ alkynyl, C₁₋₅ haloalkyl, -(C₀₋₅ alkylene)-CO-(C₁₋₅ alkyl), -(C₀₋₅ alkylene)-CO-carbocyclyl, -(C₀₋₅ alkylene)-CO-heterocyclyl, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-CO-carbocyclyl, in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-CO-heterocyclyl, in said -(C₀₋₅ alkylene)-SO₂-heterocyclyl or in said -(C₀₋₅ alkylene)-SO₂-NH-heterocyclyl are each optionally substituted with one or more groups R^{S}.

Preferably, R¹ is selected from hydrogen, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-SO₂-heterocyclyl or in said -(C₀₋₅ alkylene)-SO₂-NH-heterocyclyl are each optionally substituted with one or more groups R^{S}.

More preferably, R¹ is selected from hydrogen, -SO₂-(C₁₋₅ alkyl), -SO₂-carbocyclyl, -SO₂-heterocyclyl, -SO₂-NH₂, -SO₂-NH-(C₁₋₅ alkyl), -SO₂-NH-carbocyclyl, and -SO₂-NH-heterocyclyl, wherein the carbocyclyl moiety in said -SO₂-carbocyclyl or in said -SO₂-NH-carbocyclyl and the heterocyclyl moiety in said -SO₂-heterocyclyl or in said -SO₂-NH-heterocyclyl are each optionally substituted with one or more groups R^{S}.

Even more preferably, R¹ is selected from hydrogen, -SO₂-(C₁₋₅ alkyl), -SO₂-carbocyclyl, -SO₂-heterocyclyl, wherein the carbocyclyl moiety in said -SO₂-carbocyclyl and the heterocyclyl moiety in said -SO₂-heterocyclyl are each optionally substituted with one or more groups R^{S}.

Again more preferably, R¹ is H, -SO₂-methyl, or

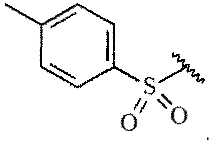

Still more preferably, R¹ is H.

In formula (I), R² is selected from hydrogen, C₁₋₅ alkyl, C₂₋₅ alkenyl, C₂₋₅ alkynyl, -(C₁₋₅ alkylene)-OH, -(C₁₋₅ alkylene)-SH, -(C₁₋₅ alkylene)-S(C₁₋₅ alkyl), -(C₁₋₅ alkylene)-NH₂, -(C₁₋₅ alkylene)-NH-C(=NH)-NH₂, -(C₁₋₅ alkylene)-COOH, - (C₁₋₅ alkylene)-CO-NH₂, phenyl, -(C₁₋₅ alkylene)-phenyl, cycloalkyl, -(C₁₋₅ alkylene)-cycloalkyl, heteroaryl, -(C₁₋₅ alkylene)-heteroaryl, heterocycloalkyl, and -(C₁₋₅ alkylene)-heterocycloalkyl, wherein said phenyl, the phenyl moiety in said -(C₁₋₅ alkylene)-phenyl, said cycloalkyl, the cycloalkyl moiety in said -(C₁₋₅ alkylene)-cycloalkyl, said heteroaryl, the heteroaryl moiety in said -(C₁₋₅ alkylene)-heteroaryl, said heterocycloalkyl, and the heterocycloalkyl moiety in said -(C₁₋₅ alkylene)-heterocycloalkyl, are each optionally substituted with one or more groups R^{S}.

Preferably, R² is selected from hydrogen, C₁₋₅ alkyl, -(C₁₋₅ alkylene)-OH, -(C₁₋₅ alkylene)-SH, -(C₁₋₅ alkylene)-S(C₁₋₅ alkyl), -(C₁₋₅ alkylene)-NH₂, -(C₁₋₅ alkylene)-NH-C(=NH)-NH₂, -(C₁₋₅ alkylene)-COOH, -(C₁₋₅ alkylene)-CO-NH₂, -(C₁₋₅ alkylene)-phenyl, and -(C₁₋₅ alkylene)-heteroaryl, wherein the phenyl moiety in said -(C₁₋₅ alkylene)-phenyl, and the heteroaryl moiety in said -(C₁₋₅ alkylene)-heteroaryl, are each optionally substituted with one or more groups R^{S}.

More preferably, R² is selected from hydrogen, methyl, isopropyl, isobutyl, sec-butyl, -CH₂OH, -CH(CH₃)OH, - CH₂SH, -CH₂CH₂-S-CH₃, -(CH₂)₃-NH₂, -(CH₂)₄-NH₂, -(CH₂)₄-NH-C(=NH)-NH₂, -(CH₂)₃-NH-C(=NH)-NH₂, - CH₂-COOH, -CH₂CH₂-COOH, -CH₂-CO-NH₂, -CH₂CH₂-CO-NH₂, -CH₂-phenyl, -CH₂-(4-hydroxyphenyl), -CH₂-(1H-imidazol-4-yl) and -CH₂-(1 H-indol-3-yl).

Even more preferably, R² is -CH₂-CO-NH₂, -CH₂CH₂-CO-NH₂, -CH₂OH, -CH(CH₃)(OH), or -CH(CH₃)(CH₂CH₃).

Even more preferably, R² is -CH₂-CO-NH₂, -CH₂CH₂-CO-NH₂, -CH₂OH, or -CH(CH₃)(OH).

Again more preferably, R² is -CH₂CH₂-CO-NH₂ or -CH₂-CO-NH₂.

Still more preferably, R² is -CH₂-CO-NH₂.

In formula (I), R³ is selected from hydrogen, -(C₁₋₅ alkylene)-NH-R^{N}, and -(C₁₋₅ alkylene)-NH-C(=NH)-NH₂, wherein R^{N} is selected from hydrogen, C₁₋₅alkyl, C₂₋₅alkenyl, C₂₋₅alkynyl, C₁₋₅haloalkyl, -(C₀₋₅ alkylene)-CO-(C₁₋₅ alkyl), -(C₀₋₅ alkylene)-CO-carbocyclyl, -(C₀₋₅ alkylene)-CO-heterocyclyl, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-CO-carbocyclyl, in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-CO-heterocyclyl, in said -(C₀₋₅ alkylene)-SO₂-heterocyclyl or in said -(C₀₋₅ alkylene)-SO₂-NH-heterocyclyl, are each optionally substituted with one or more groups R^{S}.

Preferably, R³ is selected from -(C₁₋₅ alkylene)-NH-R^{N}, and -(C₁₋₅ alkylene)-NH-C(=NH)-NH₂. More preferably, R³ is -(C₁₋₅ alkylene)-NH-R^{N}.

Concerning the definition of R^{N}, preferably R^{N} is selected from hydrogen, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-SO₂-heterocyclyl or in said -(C₀₋₅ alkylene)-SO₂-NH-heterocyclyl are each optionally substituted with one or more groups R^{S}. More preferably, R^{N} is selected from hydrogen, -SO₂-(C₁₋₅ alkyl), -SO₂-carbocyclyl, -SO₂-heterocyclyl, -SO₂-NH₂, -SO₂-NH-(C₁₋₅ alkyl), -SO₂-NH-carbocyclyl, and -SO₂-NH-heterocyclyl, wherein the carbocyclyl moiety in said -SO₂-carbocyclyl or in said -SO₂-NH-carbocyclyl and the heterocyclyl moiety in said -SO₂-heterocyclyl or in said -SO₂-NH-heterocyclyl are each optionally substituted with one or more groups R^{S}. Even more preferably, R^{N} is selected from hydrogen, -SO₂-(C₁₋₅ alkyl), -SO₂-carbocyclyl, -SO₂-heterocyclyl, wherein the carbocyclyl moiety in said -SO₂-carbocyclyl and the heterocyclyl moiety in said -SO₂-heterocyclyl are each optionally substituted with one or more groups R^{S}. Again more preferably, R^{N} is H, -SO₂-methyl, or 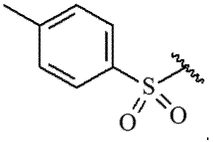 Still more preferably, R^{N} is H.

Thus, it is particularly preferred that R³ is -CH₂CH₂CH₂-NH₂, -CH₂CH₂CH₂-NH-SO₂-CH₃, or 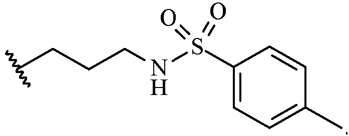 More preferably, R³ is -CH₂CH₂CH₂-NH₂.

In formula (I), R⁴ is selected from hydrogen, -C₁₋₅ alkyl, -(C₁₋₅ alkylene)-NH-C(=NH)-NH₂, -(C₁₋₅ alkylene)-CO-NH₂, -(C₁₋₅ alkylene)-(OH), and -(C₁₋₅ alkylene)-heterocyclyl, wherein the heterocyclyl moiety in said -(C₁₋₅ alkylene)-heterocyclyl is optionally substituted with one or more groups R^{S}.

Preferably, R⁴ is selected from -(C₁₋₅ alkylene)-NH-C(=NH)-NH₂, -(C₁₋₅ alkylene)-CO-NH₂, -(C₁₋₅ alkylene)-(OH), and -(C₁₋₅ alkylene)-heterocyclyl, wherein the heterocyclyl moiety in said -(C₁₋₅ alkylene)-heterocyclyl is optionally substituted with one or more groups R^{S}.

More preferably, R⁴ is selected from -(C₁₋₅ alkylene)-NH-C(=NH)-NH₂, -(C₁₋₅ alkylene)-CO-NH₂, and -(C₁₋₅ alkylene)-(OH).

Even more preferably, R⁴ is selected from -(CH₂)₄-NH-C(=NH)-NH₂, -(CH₂)₃-NH-C(=NH)-NH₂, -CH₂CH₂-CO-NH₂, - CH₂-CO-NH₂, -CH₂(OH), and -CH(CH₃)(OH).

Still more preferably, R⁴ is selected from -CH₂CH₂CH₂-NH-C(=NH)-NH₂, -CH₂(OH), and -CH(CH₃)(OH).

Again more preferably, R⁴ is -CH₂CH₂CH₂-NH-C(=NH)-NH₂ or -CH(CH₃)(OH).

Even more preferably, R⁴ is -CH₂CH₂CH₂-NH-C(=NH)-NH₂.

In formula (I), R⁵ is selected from hydrogen, C₁₋₅ alkyl, C₂₋₅ alkenyl, C₂₋₅ alkynyl, -(C₁₋₅ alkylene)-OH, -(C₁₋₅ alkylene)-SH, phenyl, -(C₁₋₅ alkylene)-phenyl, cycloalkyl, -(C₁₋₅ alkylene)-cycloalkyl, heteroaryl, -(C₁₋₅ alkylene)-heteroaryl, heterocycloalkyl, and -(C₁₋₅ alkylene)-heterocycloalkyl, wherein said phenyl, the phenyl moiety in said -(C₁₋₅ alkylene)-phenyl, said cycloalkyl, the cycloalkyl moiety in said -(C₁₋₅ alkylene)-cycloalkyl, said heteroaryl, the heteroaryl moiety in said -(C₁₋₅ alkylene)-heteroaryl, said heterocycloalkyl, and the heterocycloalkyl moiety in said -(C₁₋₅ alkylene)-heterocycloalkyl, are each optionally substituted with one or more groups R^{S}.

Preferably, R⁵ is selected from C₁₋₅ alkyl, -(C₁₋₅ alkylene)-OH, -(C₁₋₅ alkylene)-SH, phenyl, -(C₁₋₅ alkylene)-phenyl, heteroaryl, and -(C₁₋₅ alkylene)-heteroaryl, wherein said phenyl, the phenyl moiety in said -(C₁₋₅ alkylene)-phenyl, said heteroaryl, and the heteroaryl moiety in said -(C₁₋₅ alkylene)-heteroaryl, are each optionally substituted with one or more groups R^{S}.

More preferably, R⁵ is selected from C₁₋₅ alkyl, -(C₁₋₅ alkylene)-OH, -(C₁₋₅ alkylene)-phenyl, and -(C₁₋₅ alkylene)-heteroaryl, wherein the phenyl moiety in said -(C₁₋₅ alkylene)-phenyl, and the heteroaryl moiety in said -(C₁₋₅ alkylene)-heteroaryl, are each optionally substituted with one or more groups R^{S}.

Even more preferably, R⁵ is -CH(CH₃)(OH), -CH(CH₃)(CH₂CH₃), 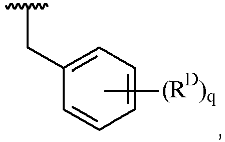 or 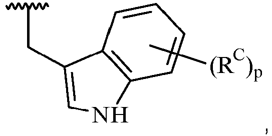 wherein R^{C} and R^{D} are each independently selected from halogen, -C₁₋₅ alkyl, and -O-C₁₋₅ alkyl and p is 0 or 1 and q is an integer from 0 to 5 (such as 0, 1, 2, 3, 4, or 5).

Again more preferably, R⁵ is

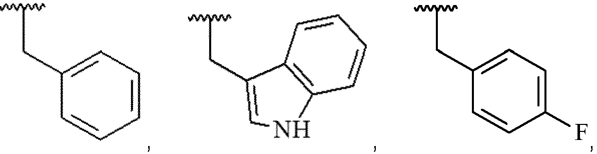

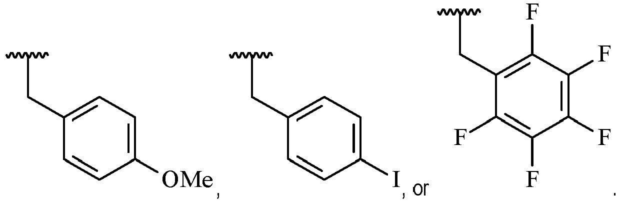

Even more preferably R⁵ is

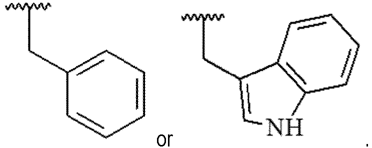

Still more preferably, R⁵ is

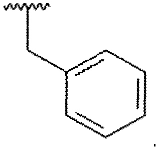

In formula (I), R⁶ is selected from -OH, -NH₂, -O(C₁₋₅ alkyl), -NH(C₁₋₅ alkyl) and N(C₁₋₅ alkyl)(C₁₋₅ alkyl).

Preferably, R⁶ is selected from -OH, -NH₂ and -OMe.

More preferably, R⁶ is -OH.

In formula (I), R^{R} is -(C₁₋₅ alkylene)-NH-C(=NH)-NH₂. Preferably, R^{R} is -CH₂CH₂CH₂-NH-C(=NH)-NH₂.

In formula (I), R^{A} and R^{B} are each individually selected from halogen, and C₁₋₅ alkyl. Preferably, R^{A} and R^{B} are each individually selected from -F, -Cl, -Br and -Me. More preferably, R^{A} and R^{B} are each individually selected from -F, - Br and -Me. Even more preferably, R^{A} and R^{B} are each individually selected from -F and -Br.

In formula (I), R^{X} and R^{Y} are each individually selected from hydrogen, C₁₋₅ alkyl, C₁₋₅ haloalkyl, and cycloalkyl. Preferably, R^{X} and R^{Y} are each individually selected from hydrogen, methyl and cyclopropyl. More preferably, R^{X} and R^{Y} are both hydrogen.

In formula (I), each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NO₂, -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -(C₀₋₃ alkylene)-carbocyclyl, and -(C₀₋₃ alkylene)-heterocyclyl, wherein the carbocyclyl moiety in said -(C₀₋₃ alkylene)-carbocyclyl and the heterocyclyl moiety in said -(C₀₋₃ alkylene)-heterocyclyl are each optionally substituted with one or more groups independently selected from C₁₋₄ alkyl, halogen, -CN, -NO₂, -OH, -O-(C₁₋₄ alkyl), -SH, -S-(C₁₋₄ alkyl), -NH₂, -NH(C₁₋₄ alkyl), -N(C₁₋₄ alkyl)(C₁₋₄ alkyl), -COOH, -COO(C₁₋₄ alkyl), -CONH₂, -CONH(C₁₋₄ alkyl), -CON(C₁₋₄ alkyl)(C₁₋₄ alkyl), -NHCO(C₁₋₄ alkyl), and -N(C₁₋₄ alkyl)-CO(C₁₋₄ alkyl).

Preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NO₂, -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), and -(C₀₋₃ alkylene)-SO-(C₁₋₅ alkyl).

More preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NO₂, -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), and -(C₀₋₃ alkylene)-O-CO-N(C₁₋₅ alkyl)-(C₁₋₅ alkyl).

Even more preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen and -(C₀₋₃ alkylene)-CN.

Again more preferably, each R^{S} is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), halogen and -CN.

Still more preferably, each R^{S} is independently selected from C₁₋₃ alkyl, -OH, -NH₂, halogen, and -CN.

Again more preferably, each R^{S} is independently selected from methyl and -F.

In formula (I), m is 0 or 1. Preferably, m is 0.

In formula (I), n is 0 or 1. Preferably, n is 0.

It is to be understood that m and n indicate the number of substituents R^{A} and R^{B}, respectively, that are bound to the respective indole ring. The corresponding substituent(s) R^{A} and R^{B} can be attached at any ring atom of the indole ring that would otherwise carry a hydrogen atom, i.e., at the phenyl ring and/or at the pyrrole ring comprised in the bicyclic indole ring. If m and/or n is 0, then the corresponding indole ring is not substituted with any group R^{A} or R^{B}, respectively, i.e. it is substituted with hydrogen instead of R^{A} or R^{B}, respectively.

As mentioned above, it is preferred that R^{R} is -CH₂CH₂CH₂-NH-C(=NH)-NH₂. Accordingly, it is preferred that the compound of formula (I) is a compound of formula (II): 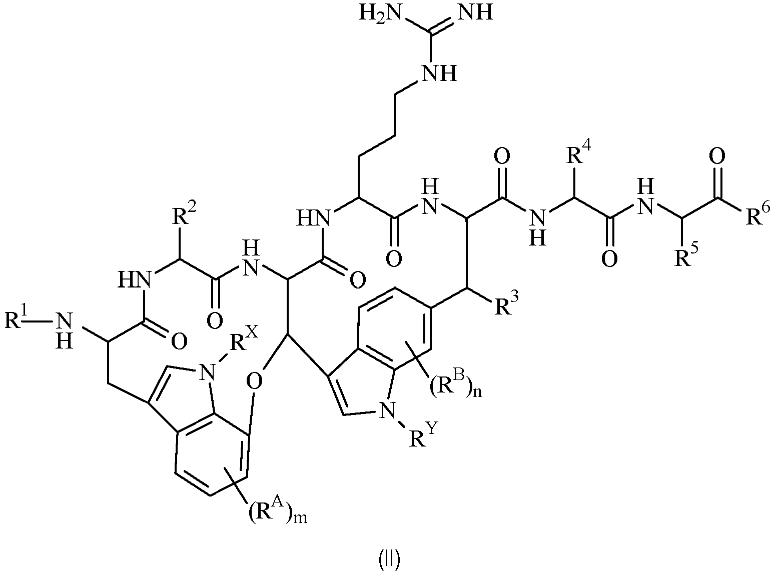 or a pharmaceutically acceptable salt thereof.

It is further particularly preferred that the configuration of the chiral centres in the compound of formula (I) is as shown in the following formula (I-a):

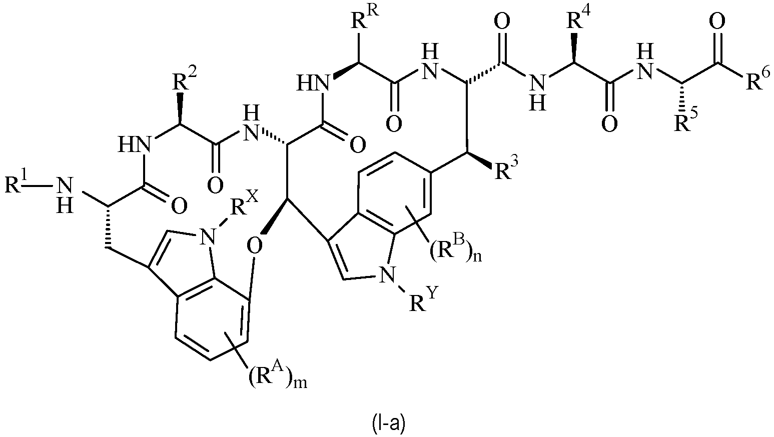

As per analogy, it is particularly preferred that the configuration of the chiral centres in the compound of formula (II) is as shown in the following formula (II-a)

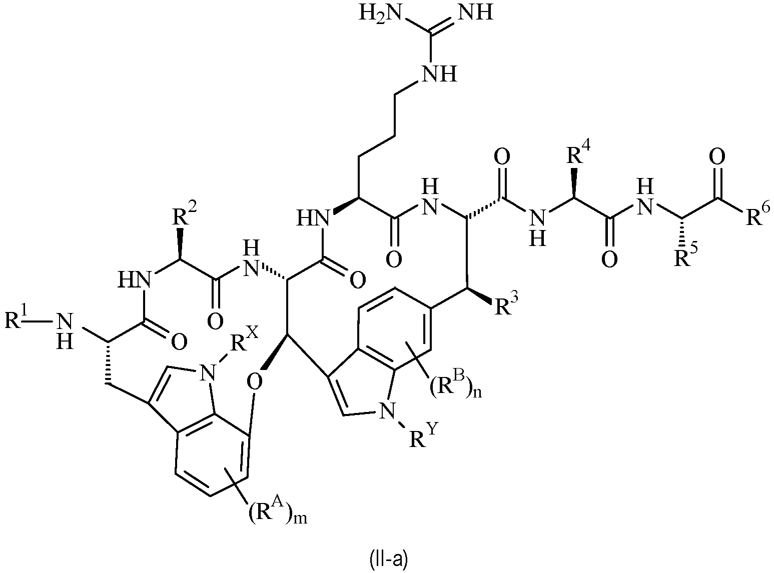

As mentioned before, R⁵ is preferably. Thus, it is preferred that the compound of formula (I) or the compound of formula (II) is a compound of formula (III): 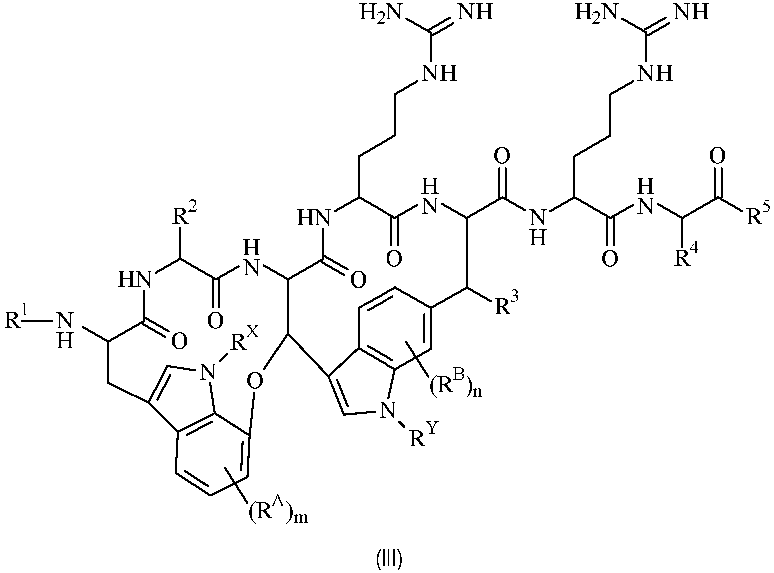 or its pharmaceutically acceptable salt.

It is particularly preferred that the configuration of the chiral centres in the compound of formula (III) is as shown in the following formula (III-a)

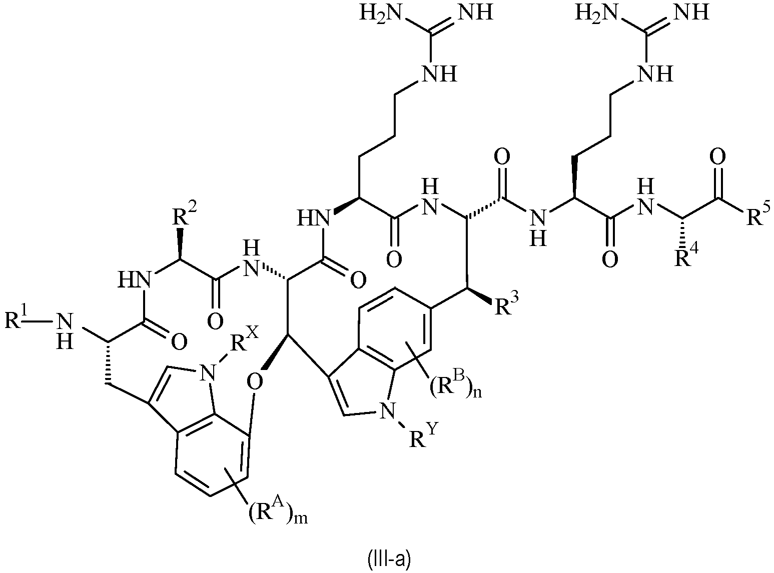

It is to be understood that any reference to a compound of formula (I) (or its pharmaceutically acceptable salt) includes individual and specific reference to a compound of formula (I-a) , a compound of formula (II), a compound of formula (II-a), a compound of formula (III) or a compound of formula (III-a) (or their respective pharmaceutically acceptable salts).

The invention is further described in the following specific embodiment. If any of the variables R¹, R², R³, R⁴, R⁵, R⁶, R^{R}, R^{N}, R^{X}, R^{Y}, R^{A}, R^{B}, R^{C,} R^{D}, R^{S}, m, n, p and q are not explicitly mentioned in the definition of the embodiment, it is to be assumed that the definition as for formula (I), including any preferred definition and specific embodiment, applies.

In a first specific embodiment of the compound of formula (I), m and n are 0 and R^{X} and R^{Y} are both hydrogen.

In a second specific embodiment of the compound of formula (I), m and/or n is 1 and R^{A} and R^{B} are each -F. It is further preferred, in this second specific embodiment that R^{X} and R^{Y} are both hydrogen.

In a third specific embodiment of the compound of formula (I), m and/or n is 1 and R^{A} and R^{B} are each -Br. It is further preferred, in this second specific embodiment that R^{X} and R^{Y} are both hydrogen.

In a fourth specific embodiment of the compound of formula (I), R¹ is -SO₂-methyl, or 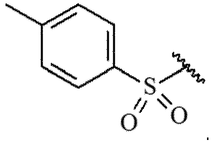 Accordingly, in this fourth specific embodiment, R¹ may be -SO₂-methyl. Alternatively, R¹ may be

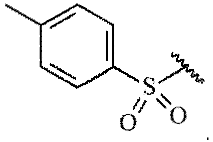

In a fifth specific embodiment of the compound of formula (I), R² is -CH(CH₃)(OH), or -CH(CH₃)(CH₂CH₃). Accordingly, in this fifth specific embodiment, R² may be -CH(CH₃)(OH). Alternatively, R² may be - CH(CH₃)(CH₂CH₃).

In a sixth specific embodiment of the compound of formula (I), R³ is -(CH₂CH₂)-NH-C(=NH)-NH₂.

In a seventh specific embodiment of the compound of formula (I), R⁴ is -CH₂(OH), or -CH(CH₃)(OH). Accordingly, in this seventh specific embodiment, R⁴ may be -CH₂(OH). Alternatively, R⁴ may be -CH(CH₃)(OH). This embodiment does not apply to the compound of formula (III) or the compound of formula (III-a).

In an eighth specific embodiment of the compound of formula (I), R⁵ is C₁₋₅ alkyl. Suitable alkyl groups include methyl, ethyl, n-propyl, isopropyl, n-butyl, isobutyl, sec-butyl, tert-butyl and n-pentyl. It is particularly preferred that, in this eighth specific embodiment, R⁵ is -CH(CH₃)(CH₂CH₃).

In a ninth specific embodiment of the compound of formula (I), R⁵ is -(C₁₋₅ alkylene)-OH. Preferably, in this ninth specific embodiment, R⁵ is -CH(CH₃)(OH).

In a tenth specific embodiment of the compound of formula (I), R⁵ is 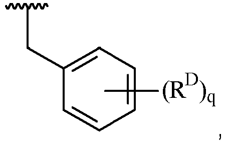 wherein R^{D} is selected from halogen, -C₁₋₅ alkyl, and -O-C₁₋₅ alkyl, and q is an integer from 0 to 5. Accordingly, q can be 0, 1, 2, 3, 4, or 5. Preferably, in this tenth specific embodiment, q is 0 or 1. More preferably, q is 0. Preferably, in this tenth specific embodiment R⁵ is selected from 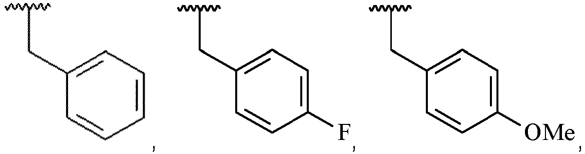 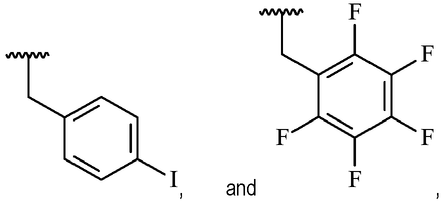 more preferably from

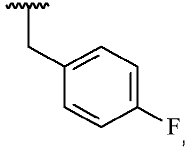

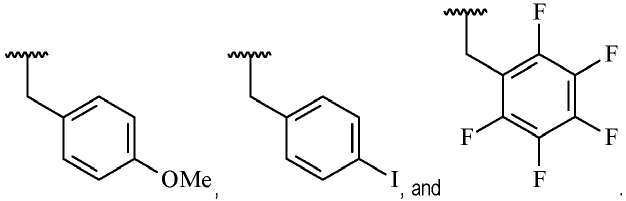

In an eleventh specific embodiment of the compound of formula (I), R⁵ is 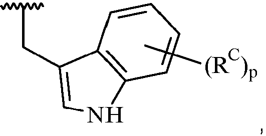 wherein R^{C} is selected from halogen, -C₁₋₅ alkyl, and -O-C₁₋₅ alkyl and p is 0 or 1. Preferably, in this eleventh specific embodiment, R⁵ is

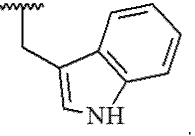

In a twelfth specific embodiment of the compound of formula (I), R⁶ is -OH.

In a thirteenth specific embodiment of the compound of formula (I), R⁶ is -NH₂.

In a fourteenth specific embodiment of the compound of formula (I), R⁶ is -OMe.

In a fifteenth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 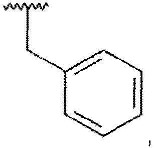 R⁶ is -OH, and R^{R} is -CH₂CH₂CH₂-NH-C(=NH)-NH₂.

In a sixteenth specific embodiment of the compound of formula (I), R¹ is hydrogen, R² is -CH₂-CO-NH₂, R³ is - CH₂CH₂CH₂-NH₂, R⁴ is -CH(CH₃)(OH), R⁵ is 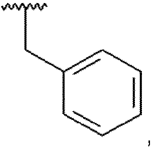 R⁶ is -OH, and R^{R} is -CH₂CH₂CH₂-NH-C(=NH)-NH₂.

In a seventeenth specific embodiment of the compound of formula (I), R¹ is hydrogen, R² is -CH₂-CO-NH₂, R³ is - CH₂CH₂CH₂-NH₂, R⁴ is -CH₂(OH), R⁵ is 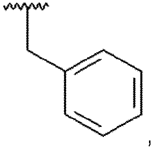 R⁶ is -OH, and R^{R} is -CH₂CH₂CH₂-NH-C(=NH)-NH₂.

In an eighteenth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 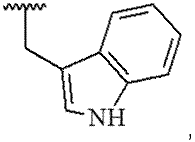 R⁶ is -OH, and R^{R} is -CH₂CH₂CH₂-NH-C(=NH)-NH₂.

In a nineteenth specific embodiment of the compound of formula (I), R¹ is hydrogen, R² is -CH₂-CO-NH₂, R³ is - CH₂CH₂CH₂-NH₂, R⁴ is -CH(CH₃)(OH), R⁵ is 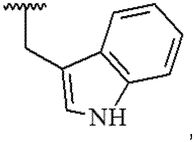 R⁶ is -OH, and R^{R} is -CH₂CH₂CH₂-NH-C(=NH)-NH₂.

In a twentieth specific embodiment of the compound of formula (I), R¹ is hydrogen, R² is -CH₂-CO-NH₂, R³ is - CH₂CH₂CH₂-NH₂, R⁴ is -CH₂(OH), R⁵ is 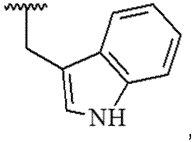 R⁶ is -OH, and R^{R} is -CH₂CH₂CH₂-NH-C(=NH)-NH₂.

In a twenty-first specific embodiment of the compound of formula (I), R¹ is hydrogen, R² is -CH₂OH, R³ is - CH₂CH₂CH₂-NH₂, R⁴ is -CH₂CH₂CH₂-NH-C(=NH)-NH₂, R⁵ is 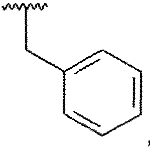 R⁶ is -OH, and R^{R} is -CH₂CH₂CH₂-NH-C(=NH)-NH₂.

In a twenty-second specific embodiment of the compound of formula (I), R¹ is hydrogen, R² is -CH₂OH, R³ is - CH₂CH₂CH₂-NH₂, R⁴ is -CH₂CH₂CH₂-NH-C(=NH)-NH₂, R⁵ is 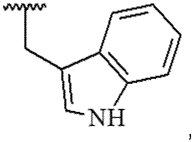 R⁶ is -OH, and R^{R} is -CH₂CH₂CH₂-NH-C(=NH)-NH₂.

In a twenty-third specific embodiment of the compound of formula (I), R¹ is hydrogen, R² is -CH(CH₃)OH, R³ is - CH₂CH₂CH₂-NH₂, R⁴ is -CH₂CH₂CH₂-NH-C(=NH)-NH₂, R⁵ is 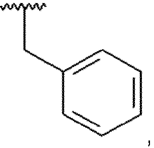 R⁶ is -OH, and R^{R} is -CH₂CH₂CH₂-NH-C(=NH)-NH₂.

In a twenty-fourth specific embodiment of the compound of formula (I), R¹ is hydrogen, R² is -CH(CH₃)OH, R³ is - CH₂CH₂CH₂-NH₂, R⁴ is -CH₂CH₂CH₂-NH-C(=NH)-NH₂, R⁵ is 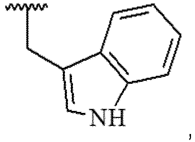 R⁶ is -OH, and R^{R} is -CH₂CH₂CH₂-NH-C(=NH)-NH₂.

In a twenty-fifth specific embodiment of the compound of formula (I), R¹ is hydrogen, R² is -CH₂-CO-NH₂, R³ is hydrogen, R⁴ is -CH₂CH₂CH₂-NH-C(=NH)-NH₂, R⁵ is 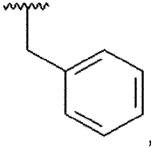 R⁶ is -OH, and R^{R} is -CH₂CH₂CH₂-NH-C(=NH)-NH₂.

In a twenty-sixth specific embodiment of the compound of formula (I), R¹ is hydrogen, R² is -CH₂-CO-NH₂, R³ is hydrogen, R⁴ is -CH₂CH₂CH₂-NH-C(=NH)-NH₂, R⁵ is 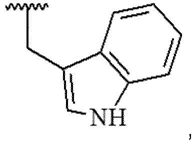 R⁶ is -OH, and R^{R} is -CH₂CH₂CH₂-NH-C(=NH)-NH₂.

In a twenty-seventh specific embodiment of the compound of formula (I), R¹ is hydrogen, R² is -CH₂-CO-NH₂, R³ is -CH₂CH₂CH₂-NH₂, R⁴ is -CH₂CH₂CH₂-NH-C(=NH)-NH₂, R⁵ is -CH₃, R⁶ is -OH, and R^{R} is -CH₂CH₂CH₂-NH-C(=NH)-NH₂.

In a twenty-eighth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is -CH(CH₃)(OH), R⁶ is -OH, and R^{R} is -CH₂CH₂CH₂-NH-C(=NH)-NH₂.

In a twenty-ninth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is -CH(CH₃)(CH₂CH₃), R⁶ is -OH, and R^{R} is -CH₂CH₂CH₂-NH-C(=NH)-NH₂.

In a thirtieth specific embodiment of the compound of formula (I), R¹ is hydrogen, R² is -CH₂-CO-NH₂, R³ is hydrogen, R⁴ is -CH₂CH₂CH₂-NH-C(=NH)-NH₂, R⁵ is 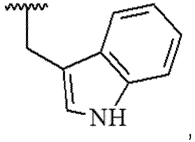 R⁶ is -OH, and R^{R} is -CH₂CH₂CH₂-NH-C(=NH)-NH₂.

In a thirty-first specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 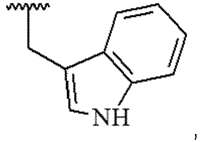 R⁶ is -OH, and R^{R} is -CH₂CH₂CH₂-NH-C(=NH)-NH₂.

In a thirty-second specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 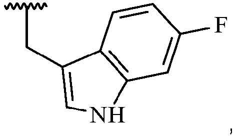 R⁶ is -OH, and R^{R} is -CH₂CH₂CH₂-NH-C(=NH)-NH₂.

In a thirty-third specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 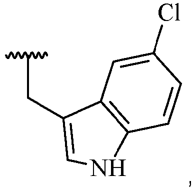 R⁶ is -OH, and R^{R} is -CH₂CH₂CH₂-NH-C(=NH)-NH₂.

In a thirty-fourth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 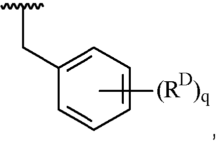 q is 1 and R^{D} is -F, R⁶ is - OH, and R^{R} is -CH₂CH₂CH₂-NH-C(=NH)-NH₂.

In a thirty-fifth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 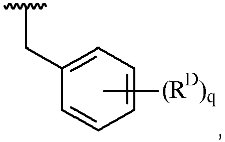 q is 1 and R^{D} is -Cl, R⁶ is - OH, and R^{R} is -CH₂CH₂CH₂-NH-C(=NH)-NH₂.

In a thirty-sixth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 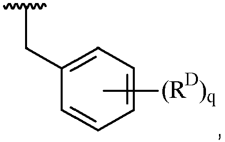 q is 1 and R^{D} is -Br, R⁶ is - OH, and R^{R} is -CH₂CH₂CH₂-NH-C(=NH)-NH₂.

In a thirty-seventh specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 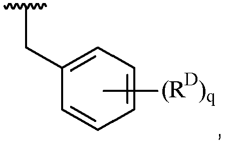 q is 1 and R^{D} is -I, R⁶ is - OH, and R^{R} is -CH₂CH₂CH₂-NH-C(=NH)-NH₂.

In a thirty-eighth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 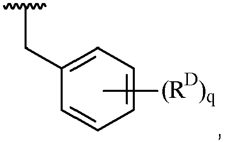 q is 1 and R^{D} is -OMe, R⁶ is -OH, and R^{R} is -CH₂CH₂CH₂-NH-C(=NH)-NH₂.

In a thirty-ninth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 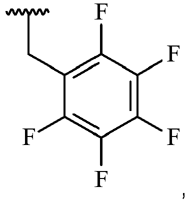 R⁶ is -OH, and R^{R} is -CH₂CH₂CH₂-NH-C(=NH)-NH₂.

In a fortieth specific embodiment of the compound of formula (I), R¹ is hydrogen, R² is -CH₂-CO-NH₂, R³ is-CH₂CH₂CH₂-NH₂, R⁴ is -CH₂CH₂CH₂-NH-C(=NH)-NH₂, R⁵ is 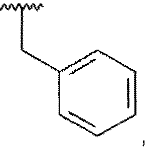 R⁶ is -OH, R^{R} is -CH₂CH₂CH₂-NH-C(=NH)-NH₂, m is 1 and R^{A} is -F.

In a forty-first specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 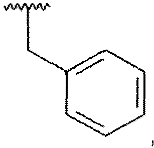 R⁶ is -OH, R^{R} is -CH₂CH₂CH₂-NH-C(=NH)-NH₂, m is 1 and R^{A} is -Cl.

In a forty-second specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 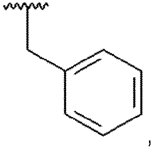 R⁶ is -OH, R^{R} is -CH₂CH₂CH₂-NH-C(=NH)-NH₂, m is 1 and R^{A} is -Br.

In a forty-third specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 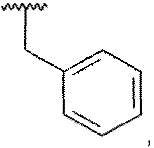 R⁶ is -OH, R^{R} is -CH₂CH₂CH₂-NH-C(=NH)-NH₂, n is 1 and R^{B} is -F.

In a forty-fourth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 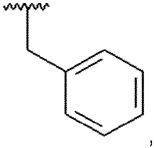 R⁶ is -OH, R^{R} is -CH₂CH₂CH₂-NH-C(=NH)-NH₂, n is 1 and R^{B} is -Cl.

In a forty-fifth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 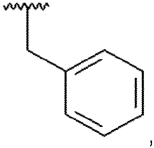 R⁶ is -OH, R^{R} is -CH₂CH₂CH₂-NH-C(=NH)-NH₂, n is 1 and R^{B} is -Br.

In a forty-sixth specific embodiment of the compound of formula (I), R¹ is -SO₂-methyl, R² is -CH₂-CO-NH₂, R³ is - CH₂CH₂CH₂-NH₂, R⁴ is -CH₂CH₂CH₂-NH-C(=NH)-NH₂, R⁵ is 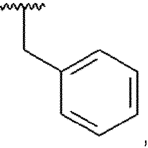 R⁶ is -OH, and R^{R} is -CH₂CH₂CH₂-NH-C(=NH)-NH₂.

In a forty-seventh specific embodiment of the compound of formula (I), R¹ is 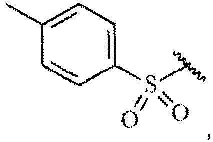 R² is -CH₂-CO-NH₂, R³ is -CH₂CH₂CH₂-NH₂, R⁴ is -CH₂CH₂CH₂-NH-C(=NH)-NH₂, R⁵ is 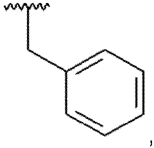 R⁶ is -OH, and R^{R} is -CH₂CH₂CH₂-NH-C(=NH)-NH₂.

In a forty-eighth specific embodiment of the compound of formula (I), R¹ is hydrogen, R² is -CH₂-CO-NH₂, R³ is - CH₂CH₂CH₂-NH-SO₂-CH₃, R⁴ is -CH₂CH₂CH₂-NH-C(=NH)-NH₂, R⁵ is 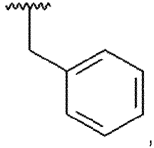 R⁶ is -OH, and R^{R} is -CH₂CH₂CH₂-NH-C(=NH)-NH₂.

In a forty-ninth specific embodiment of the compound of formula (I), R¹ is hydrogen, R² is -CH₂-CO-NH₂, R³ is 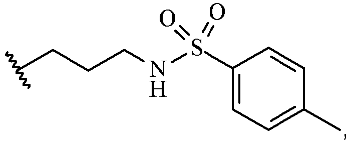 R⁴ is -CH₂CH₂CH₂-NH-C(=NH)-NH₂, R⁵ is 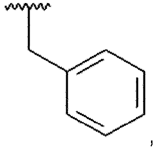 R⁶ is -OH, and R^{R} is -CH₂CH₂CH₂-NH-C(=NH)-NH₂.

In a fiftieth specific embodiment of the compound of formula (I), R¹ is hydrogen, R² is -CH₂-CO-NH₂, R³ is - CH₂CH₂CH₂-NH₂, R⁴ is -CH₂CH₂CH₂-NH-C(=NH)-NH₂, R⁵ is 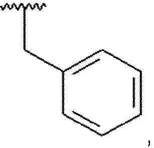 R⁶ is -OH, and R^{R} is -CH₂CH₂CH₂-NH-C(=NH)-NH₂.

In a fifty-first specific embodiment of the compound of formula (I), R² is -CH₂-CO-NH₂, -CH₂CH₂-CO-NH₂, -CH₂OH, or -CH(CH₃)(OH).

In a fifty-second specific embodiment of the compound of formula (I), R³ is -CH₂CH₂CH₂-NH₂, or -CH₂CH₂-NH-C(=NH)-NH₂

Examples of the compounds of formula (I) include, in particular, darobactin R, darobactin R1 and darobactin R2 as well as pharmaceutically acceptable salts and solvates of either of these compounds:

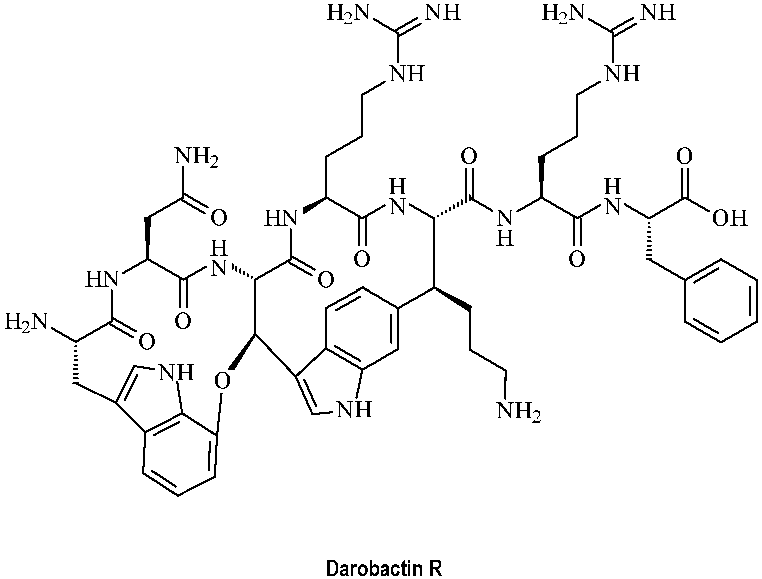

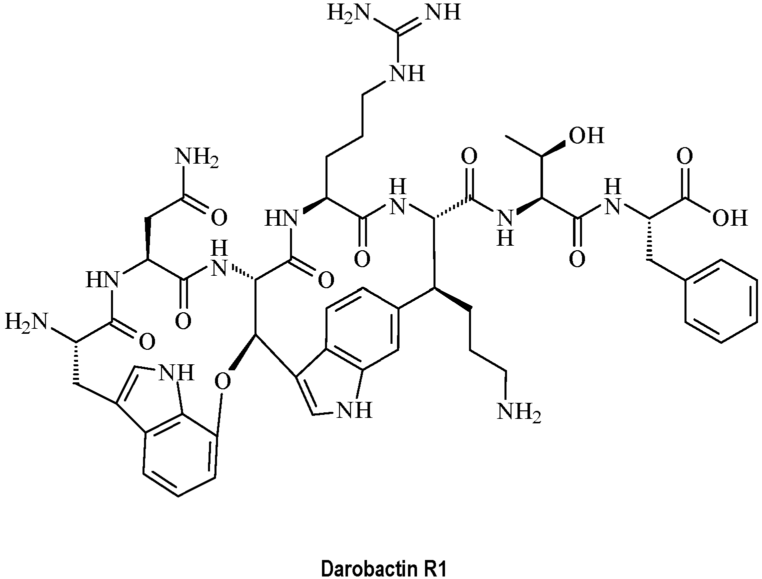

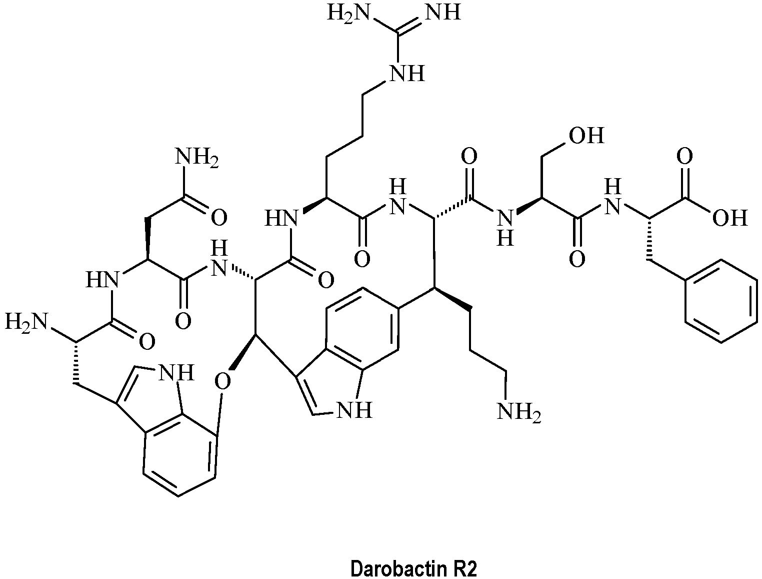

Most preferably, the compound of formula (I) is darobactin R: 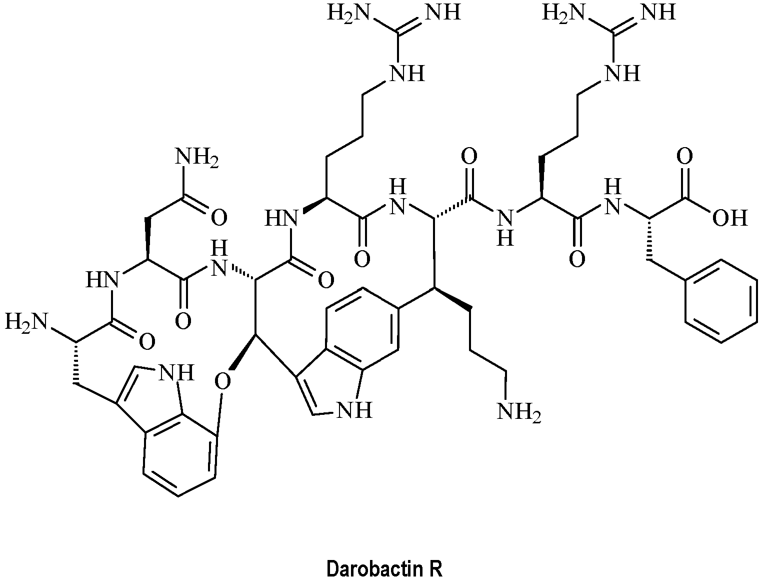 or its pharmaceutically acceptable salt.

Further preferred compounds of formula (I) include the compounds described in any one of specific embodiments from 15^{th} specific embodiment to 50^{th} specific embodiment.

The compounds of formula (I) can be prepared in accordance with, or in analogy to, the synthetic routes described in the examples section (including synthetic routes involving recombinant production of the compounds in the host organisms).

The following definitions apply throughout the present specification and the claims, unless specifically indicated otherwise.

The term "hydrocarbon group" refers to a group consisting of carbon atoms and hydrogen atoms.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₆ alkyl" denotes an alkyl group having 1 to 6 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₆ alkenyl" denotes an alkenyl group having 2 to 6 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₆ alkynyl" denotes an alkynyl group having 2 to 6 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₆ alkylene" denotes an alkylene group having 1 to 6 carbon atoms, and the term "C₀₋₆ alkylene" indicates that a covalent bond (corresponding to the option "C₀ alkylene") or a C₁₋₆ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkenyl or cycloalkyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkenyl or heterocycloalkyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members (e.g., cyclopropyl or cyclohexyl).

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, 1,1-dioxothianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-CI), bromo (-Br), or iodo (-I).

As used herein, the term "nitro" refers to a group -NO₂.

The terms "bond" and "covalent bond" are used herein synonymously, unless explicitly indicated otherwise or contradicted by context.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

It is to be understood that wherever numerical ranges are provided/disclosed herein, all values and subranges encompassed by the respective numerical range are meant to be encompassed within the scope of the invention. Accordingly, the present invention specifically and individually relates to each value that falls within a numerical range disclosed herein, as well as each subrange encompassed by a numerical range disclosed herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

As understood herein, "MIC₉₀" refers to the MIC value at which ≥90% of the individual isolates of a test population of a given species, for example, a representative cohort of clinical *E.coli* isolates, is inhibited. More specifically, the MIC₉₀ value for a specific species, is obtained by determining the MICs of reasonably large population (n > 100) of isolates of that species using standard methods, such as micro-broth dilution turbidity tests (for example, as described herein). If data distribution is unimodal, results are sorted from lowest to highest MIC. The MIC of the isolate at rank n × 0.9 (the 90^{th} percentile) represents the MIC₉₀ value. Further information in this regard can be found in Schwarz et al., Journal of Antimicrobial Chemotherapy, Volume 65, Issue 4, April 2010, Pages 601-604, https://doi.org/10.1093/jac/dkq037 .

The scope of the invention embraces all pharmaceutically acceptable salt forms of the compounds of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (I) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) is a hydrochloride salt. Accordingly, it is preferred that the compound of formula (I), including any one of the specific compounds of formula (I) described herein, is in the form of a fumarate salt, a maleate salt, an oxalate salt, a malate salt, a tartrate salt, and a mesylate salt.

The present invention also specifically relates to the compound of formula (I), including any one of the specific compounds of formula (I) described herein, in non-salt form.

Moreover, the scope of the invention embraces the compounds of formula (I) in any solvated form, including, e.g., solvates with water (i.e., as a hydrate) or solvates with organic solvents such as, e.g., methanol, ethanol, isopropanol, acetic acid, ethyl acetate, ethanolamine, DMSO, or acetonitrile. All physical forms, including any amorphous or crystalline forms (i.e., polymorphs), of the compounds of formula (I) are also encompassed within the scope of the invention. It is to be understood that such solvates and physical forms of pharmaceutically acceptable salts of the compounds of the formula (I) are likewise embraced by the invention.

Furthermore, the compounds of formula (I) may exist in the form of different isomers, in particular stereoisomers (including, e.g., geometric isomers (or cis/trans isomers), enantiomers and diastereomers) or tautomers (including, in particular, prototropic tautomers, such as keto/enol tautomers or thione/thiol tautomers). All such isomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates and non-racemic mixtures). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. In particular, the compounds of formula (I) generally comprise L-amino acids or derivatives thereof. Preferably, all the amino acids are L-amino acids, unless indicated to the contrary. However, one or more of the amino acids or derivatives thereof comprised in the compounds of formula (I) may be present as R-amino acids or derivatives thereof. Consequently, compounds of formula (I) are preferably characterizing by stereochemistry as shown in formula (I-a):

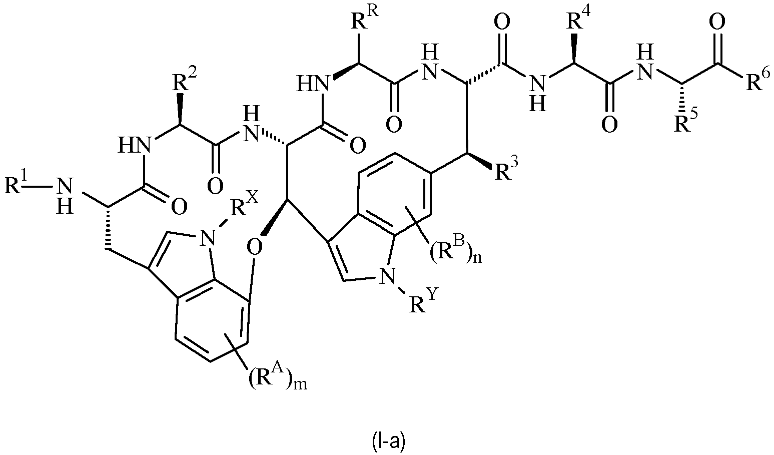

Likewise, compounds of formula (II) are preferably characterized by the stereochemistry as shown in formula (II-a)

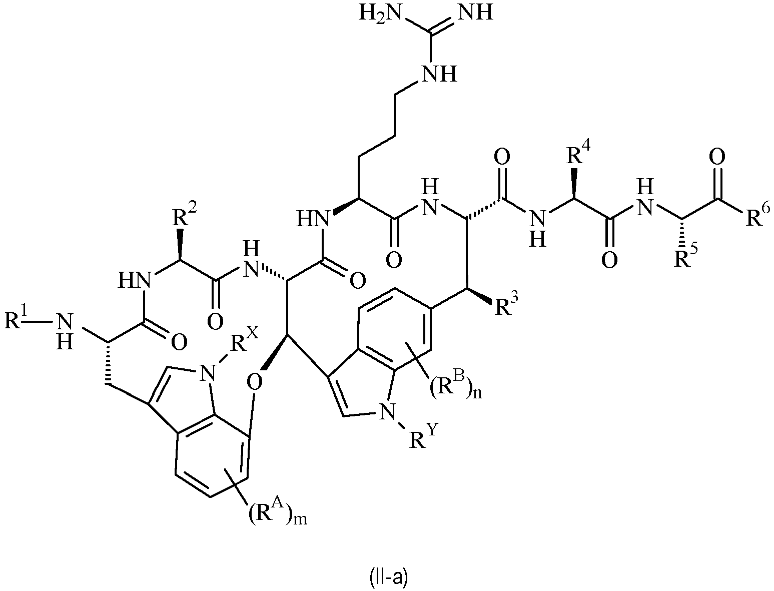

Likewise, compounds of formula (III) are preferably of the following stereochemistry (III-a)

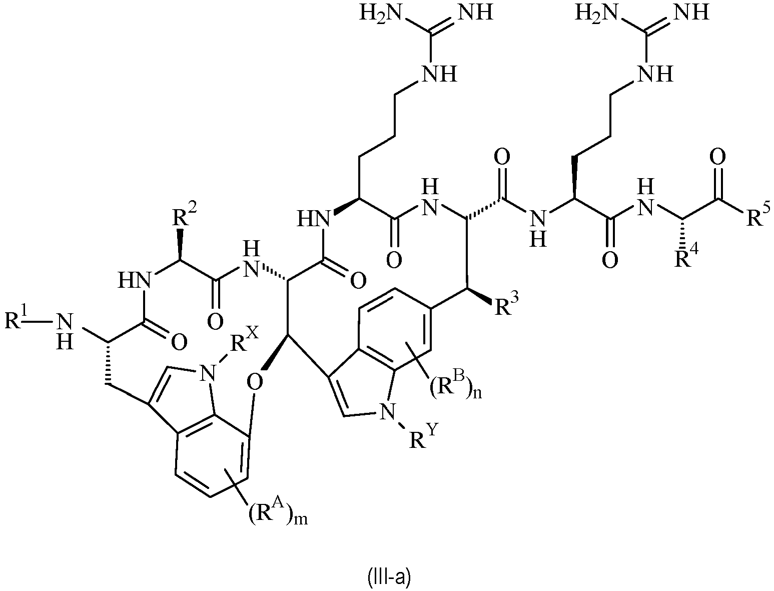

The present invention further encompasses any tautomers of the compounds of formula (I). It will be understood that some compounds may exhibit tautomerism. In such cases, the formulae provided herein expressly depict only one of the possible tautomeric forms. The formulae and chemical names as provided herein are intended to encompass any tautomeric form of the corresponding compound and not to be limited merely to the specific tautomeric form depicted by the drawing or identified by the name of the compound.

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., ²H; also referred to as "D"). Accordingly, the invention also embraces compounds of formula (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces compounds of formula (I), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers, trackers or imaging probes in positron emission tomography (PET). The invention thus includes (i) compounds of formula (I), in which one or more fluorine atoms (or, e.g., all fluorine atoms) are replaced by ¹⁸F atoms, (ii) compounds of formula (I), in which one or more carbon atoms (or, e.g., all carbon atoms) are replaced by ¹¹C atoms, (iii) compounds of formula (I), in which one or more nitrogen atoms (or, e.g., all nitrogen atoms) are replaced by ¹³N atoms, (iv) compounds of formula (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula (I), in which one or more bromine atoms (or, e.g., all bromine atoms) are replaced by ⁷⁷Br atoms, (vii) compounds of formula (I), in which one or more iodine atoms (or, e.g., all iodine atoms) are replaced by ¹²⁰I atoms, and (viii) compounds of formula (I), in which one or more iodine atoms (or, e.g., all iodine atoms) are replaced by ¹²⁴I atoms. In general, it is preferred that none of the atoms in the compounds of formula (I) are replaced by specific isotopes.

The present invention relates to a pharmaceutical composition comprising a compound of formula (I) or a pharmaceutically acceptable salt thereof of the present invention as defined herein, and a pharmaceutically acceptable excipient. The present invention further relates to the said pharmaceutical composition comprising a compound of formula (I) or a pharmaceutically acceptable salt thereof of the present invention, and a pharmaceutically acceptable excipient for use in therapy.

The compounds and/or chemical entities provided herein may be administered as compounds *per se* or may be formulated as medicaments. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor^{®} HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-a-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

The pharmaceutical compositions may also comprise one or more preservatives, particularly one or more antimicrobial preservatives, such as, e.g., benzyl alcohol, chlorobutanol, 2-ethoxyethanol, m-cresol, chlorocresol (e.g., 2-chloro-3-methyl-phenol or 4-chloro-3-methyl-phenol), benzalkonium chloride, benzethonium chloride, benzoic acid (or a pharmaceutically acceptable salt thereof), sorbic acid (or a pharmaceutically acceptable salt thereof), chlorhexidine, thimerosal, or any combination thereof.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) or the pharmaceutically acceptable salts thereof or the above described pharmaceutical compositions comprising the compound of formula (I) or the pharmaceutically acceptable salt thereof may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, or vaginal administration.

If said compounds or pharmaceutically acceptable salts or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutically acceptable salts or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

For oral administration, the compounds, the pharmaceutically acceptable salts or the pharmaceutical compositions are preferably administered by oral ingestion, particularly by swallowing. The compounds or pharmaceutical compositions can thus be administered to pass through the mouth into the gastrointestinal tract, which can also be referred to as "oral-gastrointestinal" administration.

Alternatively, said compounds, pharmaceutically acceptable salts or pharmaceutical compositions can be administered in the form of a suppository or pessary, or may be applied topically in the form of a gel, hydrogel, lotion, solution, cream, ointment or dusting powder. The compounds of the present invention may also be dermally or transdermally administered, for example, by the use of a skin patch.

Said compounds, pharmaceutically acceptable salts or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides, copolymers of L-glutamic acid and gamma-ethyl-L-glutamate, poly(2-hydroxyethyl methacrylate), ethylene vinyl acetate, or poly-D-(-)-3-hydroxybutyric acid. Sustained-release pharmaceutical compositions also include liposomally entrapped compounds. The present invention thus also relates to liposomes containing a compound of the invention or a pharmaceutically acceptable salt thereof.

Said compounds, pharmaceutically acceptable salts or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) or the pharmaceutically acceptable salt thereof for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to an emulsification/spray drying process.

For topical application to the skin, said compounds, pharmaceutically acceptable salts or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water. The present invention thus relates to the compounds, pharmaceutically acceptable salts or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. Preferred routes of administration are oral administration or parenteral administration. For each of the compounds, salts or pharmaceutical compositions provided herein, it is particularly preferred that the respective compound or pharmaceutical composition is to be administered orally (particularly by oral ingestion).

The compound of formula (I) or a pharmaceutical composition comprising the compound of formula (I) can be administered in monotherapy (e.g., without concomitant administration of any further therapeutic agents or, in particular, without concomitant administration of any further anticancer drugs). However, the compound of formula (I) or a pharmaceutical composition comprising the compound of formula (I) can also be administered in combination with one or more further therapeutic agents. If the compound of formula (I) is used in combination with a second therapeutic agent active against the same disease or condition (e.g., a further antibiotic drug), the dose of each compound may differ from that when the corresponding compound is used alone, in particular, a lower dose of each compound may be used. The combination of the compound of formula (I) with one or more further therapeutic agents may comprise the simultaneous/concomitant administration of the compound of formula (I) and the further therapeutic agent(s) (either in a single pharmaceutical formulation or in separate pharmaceutical formulations), or the sequential/separate administration of the compound of formula (I) and the further therapeutic agent(s). If administration is sequential, either the compound of formula (I) according to the invention or the one or more further therapeutic agents may be administered first. If administration is simultaneous, the one or more further therapeutic agents may be included in the same pharmaceutical formulation as the compound of formula (I), or they may be administered in one or more different (separate) pharmaceutical formulations.

Preferably, the one or more further therapeutic agents to be administered in combination with a compound of the present invention are antibiotic drugs. The antibiotic drug(s) to be administered in combination with a compound of formula (I) according to the invention may, e.g., be selected from: a glycopeptide, a rifamycin, a sulfonamide, a beta-lactam, a tetracycline, an amphenicol (such as chloramphenicol), an aminoglycoside, a macrolide, a streptogramin, a quinolone, a fluoroquinolone, an oxazolidinone and a lipopeptide. Specific examples of antibiotics include, but are not limited to, tetracycline, colistin (polymyxin B) tigecycline, minocycline, oxazolidinone antibacterial agents, aminoglycoside antibacterial agents, quinolone antibacterial agents, vancomycin, teicoplanin, eremomycin, oritavancin, chloroeremomycin, and daptomycin.

In addition or alternatively, one or more further therapeutic agents selected from an antifungal agent, an anthelmintic agent, an antimalarial agent, an antiprotozoal agent, an antituberculosis agent, and an antiviral agent may also be administered in combination with the compound of the present invention.

The antifungal agent(s) to be administered in combination with a compound of formula (I) according to the invention may, e.g., be selected from: terbinafine, itraconazole, ketoconazole, fluconazole, derivatives of fluconazole, oxiconazole, sulconazole, clotrimazole, miconazole, econazole, azanidazole, bifonazole, butoconazole, chlormidazole, fenticonazole, imazalil, isoconazole, neticonazole, sertaconazole, tioconazole, naftifine, griseofulvin, amorolfine, and sodium pyrithione.

The anthelmintic agent(s) to be administered in combination with a compound of formula (I) according to the invention may, e.g., be selected from: Albendazole, Ivermectin, Mebendazole, Nitazoxanide, Pentamidine, Praziquantel, Pyrantel, Thiabendazole, and Triclabendazole.

The antimalarial agent(s) to be administered in combination with a compound of formula (I) according to the invention may, e.g., be selected from: Artemisinin, Amodiaquine, Atovaquone, Chloroquine, Mefloquine, Mepacrine, Primaquine, Proguanil, Quinine, Sulfadoxine-Pyrimethamine, and Tafenoquine.

The antiprotozoal agent(s) to be administered in combination with a compound of formula (I) according to the invention may, e.g., be selected from: Eflornithine, Furazolidone, Hydroxychloroquine, Melarsoprol, Metronidazole, Nifursemizone, Nitazoxanide, Ornidazole, Paromomycin sulfate, Pentamidine, Pyrimethamine, Quinapyramine, Ronidazole, and Tinidazole.

The antituberculosis agent(s) to be administered in combination with a compound of formula (I) according to the invention may, e.g., be selected from: Bedaquiline, Capreomycin, Cycloserine, Ethambutol, Ethionamide, Isoniazid, Pretomanid, Pyrazinamide, Rifabutin, Rifampin, Rifapentine, and Streptomycin.

The antiviral agent(s) to be administered in combination with a compound of formula (I) according to the invention may, e.g., be selected from: Fostemsavir, Enfuvirtide, Maraviroc, Bictegravir, Cabotegravir, Dolutegravir, Elvitegravir, Raltegravir, Ibalizumab, Delavirdine, Doravirine, Efavirenz, Etravirine, Nevirapine, Rilpivirine, Abacavir, Didanosine, Emtricitabine, Lamivudine, Remdesivir, Stavudine, Tenofovir, Zidovudine, Amprenavir, Atazanavir, Darunavir, Fosamprenavir, Indinavir, Lopinavir, Nelfinavir, Ritonavir, Saquinavir, Tipranavir, Alpha Interferon, Adefovir, Emtricitabine, Entecavir, Lamivudine, Telbivudine, Tenofovir, Daclatasvir, Elbasvir, Ledipasvir, Ombitasvir, Pibrentasvir, Velpatasvir, Dasabuvir, Sofosbuvir, Asunaprevir, Boceprevir, Glecaprevir, Grazoprevir, Paritaprevir, Simeprevir, Telaprevir, Voxilaprevir, Epclusa, Harvoni, Mavyret, Technive, Viekira Pak, Vosevi, Zepatier, Acyclovir, Cidofovir, Famciclovir, Foscarnet, Ganciclovir, Letermovir, Maribavir, Valacyclovir, Valganciclovir, Amantadine, Baloxavir, Oseltamivir, Peramivir, Rimantadine, Zanamivir, Molnupiravir, Paxlovid, Brincidofovir, and Tecovirimat.

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the compound of the present invention (i.e., the compound of formula (I) or a pharmaceutically acceptable salt or solvate thereof) or the further therapeutic agent(s) may be administered first. When administration is simultaneous, the combination may be administered either in the same pharmaceutical composition or in different pharmaceutical compositions. When combined in the same formulation, it will be appreciated that the two or more compounds must be stable and compatible with each other and the other components of the formulation. When formulated separately, they may be provided in any convenient formulation.

Yet, the compounds of formula (I) can also be used in monotherapy, particularly in the monotherapeutic treatment or prevention of treatment or prevention of a bacterial infectious disease, preferably caused by a Gram-negative bacterium (i.e., without administering any other antimicrobial agents until the treatment with the compound(s) of formula (I) is terminated). Accordingly, the invention also relates to a compound of formula (I) or a pharmaceutically acceptable salt or solvate thereof, or a pharmaceutical composition comprising any of the aforementioned entities in combination with a pharmaceutically acceptable excipient, for use in the monotherapeutic treatment or prevention of a bacterial infectious disease, preferably caused by a Gram-negative bacterium.

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of the compounds according to the invention for oral administration to a human (of approximately 70 kg body weight) may be 0.05 to 2000 mg, preferably 0.1 mg to 1000 mg, of the active ingredient per unit dose. The unit dose may be administered, e.g., 1 to 3 times per day. The unit dose may also be administered 1 to 7 times per week, e.g., with not more than one administration per day. It will be appreciated that it may be necessary to make routine variations to the dosage depending on the age and weight of the patient/subject as well as the severity of the condition to be treated. The precise dose and also the route of administration will ultimately be at the discretion of the attendant physician or veterinarian.

The invention further relates to the compound of formula (I) or the pharmaceutically acceptable salt thereof or to the for use as a medicament. As understood herein the use as a medicament is meant as the use in the treatment of a disease.

As used herein, the term "treatment" (or "treating") in relation to a disease or disorder refers to the management and care of a patient for the purpose of combating the disease or disorder, such as to reverse, alleviate, inhibit or delay the disease or disorder, or one or more symptoms of such disease or disorder. It also refers to the administration of a compound or a composition for the purpose of preventing the onset of symptoms of the disease or disorder, alleviating such symptoms, or eliminating the disease or disorder. Preferably, the "treatment" is curative, ameliorating or palliative.

The term "prevention" of a disorder or disease as used herein (e.g., "prevention" of an infections disease) is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of a compound of the present invention before any clinical and/or pathological symptoms are diagnosed or determined or can be diagnosed or determined by the attending physician.

It is to be understood that the present invention specifically relates to each and every combination of features and embodiments described herein, including any combination of general and/or preferred features/embodiments. In particular, the invention specifically relates to each combination of meanings (including general and/or preferred meanings) for the various groups and variables comprised in formula (I).

The compounds of formula (I) or the pharmaceutically acceptable salts thereof, or the pharmaceutical compositions of the present invention are useful in the in the treatment or prevention of a bacterial infectious disease, preferably caused by a Gram-negative bacterium.

The subject or patient to be treated in accordance with the present invention may be an animal (e.g., a non-human animal). Preferably, the subject/patient is a mammal. More preferably, the subject/patient is a human (e.g., a male human or a female human) or a non-human mammal. Most preferably, the subject/patient to be treated in accordance with the invention is a human.

In this specification, a number of documents including patent applications, scientific literature and manufacturers' manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

As understood herein, the bacterial infectious disease to be treated with the compound of formula (I) is preferably caused by one or more bacteria selected from Pseudomonas aeruginosa, Klebsiella pneumoniae, Acinetobacter baumannii, Neisseria gonorrhoeae, Chlamydia trachomatis, Shigella sonnei, Salmonella enterica Typhimurium LT2, Enterobacter cloacae, Bifidobacterium longum, Bacteroides fragilis, Lactobacillus reuteri, Enterococcus faecalis, Yersinia pestis, Pseudomonas, fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Enterobacter aerogenes, Enterobacter spp., Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter calcoaceticus, Acinetobacter haemolyticus, Yersinia enterocolitica, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria meningitidis, Kingella, Moraxella, Gardnerella vaginalis, Bacteroides distasonis, Bacteroides 3452A homology group, Bacteroides vulgatus, Bacteroides ovalus, Bacteroides thetaiotaomicron, Bacteroides uniformis, Bacteroides eggerthii, and Bacteroides splanchnicus.

The invention will now be described by reference to the following examples which are merely illustrative and are not to be construed as any limitation of the scope of the present invention.

### EXAMPLES

### Example 1: Production, isolation and biotechnological derivatization of darobactin type BamA Inhibitors

For the production of darobactin type BamA Inhibitors (DARs), the dar operon of a donor organism (such as Photorhabdus khanii, Yersinia pestis, Pseudoalteromonas luteoviolacea or other) was expressed in total (darABCDEFGH) or in part (darA and darE being the minimally required genes for darobactin type BamA Inhibitor biosynthesis) in a suited procaryotic or eukaryotic host (such as Escherichia coli, Vibrio natriegens, Pichia pastoris or other). Therefore, the operon or its individual genes were placed under the control of one or more constitutive or inducible promotors suited for the respective host (such as T7, LAC, ARA, AOX1 or other). The expression construct for the production can be situated outside of the chromosomal DNA on a plasmid, fosmid, or other suited genetic constructs or situated on the chromosomal DNA.

For the generation of derivatives, the 21 nucleotides of darA coding for the seven core amino acids (WT translated amino acid sequence of darobactin A: NH2-WNWSKSF-COOH) (SEQ ID NO.: 3) can be freely modified to code for any other amino acid sequence by standard molecular biological methodology. Specifically, to obtain a derivative with superior in-vitro and in-vivo potency against key pathogens such as E. coli, Klebsiella pneumoniae or Pseudomonas aeruginosa with the amino acid sequence NH2-WNWRKRF-COOH (preliminarily termed "darobactin R" or "DAR R"; C53H69N17O10; see Fig. 4) (SEQ ID NO.: 1), the darA core coding region was modified to yield the translated amino acid sequence NH2-WNWRKRF-COOH (SEQ ID NO.: 1), which in turn was synthesized and modified by DarE and host endogenous proteases to the respective molecule.

A such engineered producer strain was subsequently cultivated in a suited expression medium (such as LB, TSB, M9 or other) in the presence of a selection marker (such as kanamycin, ampicillin, chloramphenicol or other) to maintain the stability of the genetic construct if needed. If inducible promotors are utilized to drive the expression, a suited inducer was added to the culture from the start or after a uninduced growth phase. After fermentative production, the whole broth was either extracted directly using liquid-liquid extraction (using suited solvents such as ethyl acetate, 1-butanol or another suitable solvent or solvent mixture), or the cells were harvested and the supernatant subjected to liquid-liquid and/or solid phase extraction, creating a concentrated crude extract containing the desired product. This crude extract was further purified using chromatography methods such as medium pressure normal-phase or reverse phase chromatography, ion-exchange chromatography, size exclusion chromatography or other suited methodology. Final purification was performed on C-18 reverse-phase high performance liquid chromatography (HPLC) utilizing suited mobile phases such as methanol/H2O or acetonitrile/H2O with or without acidification or other suited methodology.

Variants bearing non-canonical amino acid residues, such as derivatives of phenylalanine, tryptophan and/or tyrosine, could be prepared according to strategies outlined by Wang *et al.* (Wang, Y. S., Fang, X., Wallace, A. L., Wu, B. & Liu, W. R. A rationally designed pyrrolysyl-tRNA synthetase mutant with a broad substrate spectrum. J. Am. Chem. Soc. 134, 2950-2953 (2012)) and Wang *et al.* (Wang L., Brock A., Herberich B and Schulz P. G., Expanding the Genetic Code of Escherichia coli. Science 292, 498-500 (2001)). As outlined above, the minimal biosynthetic gene cluster for heterologous darobactin production comprises darA, which encodes the precursor peptide featuring the core sequence of the heptapeptidic product, and darE, which encodes a radical S-adenosylmethionine enzyme (RaS) responsible for macrocyclization of the peptide. Modification of the core peptide sequence in darA yields new darobactin-peptides. An amber stop codon suppression technique can be used to conduct the targeted introduction of non-canonical amino acids into the bicyclic heptapeptide. Accordingly, in order to replace C-terminal phenylalanine with a non-canonical residue, the triplet coding for the C-terminal phenylalanine F7 in the core peptide region of the native darA gene is mutated to the amber stop codon TAG. A pyrrolysyl-tRNA synthase-pair from *Methanosarcina Mazei,* which was engineered by Wang *et al.* (Wang, Y. S., Fang, X., Wallace, A. L., Wu, B. & Liu, W. R. A rationally designed pyrrolysyl-tRNA synthetase mutant with a broad substrate spectrum. J. Am. Chem. Soc. 134, 2950-2953 (2012)) to accept phenylalanine derivatives with large substituents in the para position is used. The modified darA plus darE are co-expressed on one plasmid and the pyrrolysyl-tRNA synthase-pair on a second plasmid in *E. coli* BL21. The cultivation is done in minimal medium supplemented with the respective non-canonical phenylalanine (in absence of regular phenylalanine). This way, biosynthesis of new darobactin derivatives with an altered F7 residue can be obtained. Similar protocol is applicable for obtaining non-canonical amino acid residues at other positions.

For the generation of halogenated derivatives, such as derivatives modified at one or both of core tryptophan residues, suitable enzymes, as the FAD-dependent halogenase encoded in the BGCs of *Pseudoalteromonas luteoviolacea* H33 and H33S, can be used in vivo or in vitro. As it is known to the person skilled in the art, the position of the halogen within the aromatic ring can be altered by the usage of different halogenases, which are catalyzing halogenation at a specific position.

### Example 2: In vitro microbiology assays

### Minimum inhibitory concentration (MIC) measurements:

The minimum inhibitory concentration of the purified darobactin R (DAR R) and reference darobactins (DAR B, DAR B9 and DAR A) were determined by microbroth dilution assays. Overnight cultures of the test strains were cultivated at 37°C and 180 rpm in cation adjusted Mueller Hinton II broth. Before the bacterial cultures were distributed to 96-well assay plates, the cell densities of the respective cultures were adjusted to 5 × 10⁵ cells/mL each. All compounds were dissolved in water and tested in a triplicated 12-point dilution series. In addition, dilution series of ceftazidime (CAZ), ciprofloxacin (CIP) and gentamicin (GEN) were used as positive control for each strain on each assay plate. Bacterial suspension without test compound or positive control was used as negative control e.g. growth control. Medium background was averaged from 5 replicates. Assay plates were incubated for 18h at 180 rpm and 85% relative humidity before the turbidity of all wells was determined with a microplate spectrophotometer at 600 nm (LUMIstar^{®} Omega BMG Labtech). Growth inhibition was calculated relative to the absorption of the controls.

**Table 1: MICs of darobactin R and reference compounds against Gram-negative bacteria. Values given in µg/mL. Pathogens were screened in growth medium supplemented with human serum instead of regular medium (cation adjusted Mueller Hinton II broth).**

| | | **DAR R** | **DAR B** | **DAR B9*** | **DAR A** | **CAZ** | **CIP** | **GEN** |
|---|---|---|---|---|---|---|---|---|
| *E. coli* | ATCC 35218 | 0.06 | 1 | 1 | 8-4 | 0.25-0.125 | 0.008 | 2 |
| | ATCC 25922 | 0.06 | 1-05 | 1 | | 0.5-0.25 | 0.008 | 1-0.5 |
| | ATCC25922 ΔToIC | 0.06 | 0.5-0.25 | 0.25 | | 0.5-0.25 | 0.004-0.001 | 0.5 |
| | NRZ14408 | 0.06 | 1-0.5 | | 4 | 32-16 | >0.5 | >64 |
| | K0416 | 0.06-0.03 | 1-0.5 | 1 | 4 | 64 | >0.5 | 4 |
| | Survcare052 | 0.25-0.125 | 1 | 2 | 8-4 | >64 | >0.5 | 0.5 |
| *P. aeruginosa* | PA01 | 0.25 | 2-1 | 1 | 4-2 | 2 | 0.25 | 2 |
| | PA103 | 2 | 8 | 4 | | 8 | 0.06 | 2 |
| | ATCC27853 | 4 | 8 | 8 | >64 | 4 | 0.125 | 2 |
| | EXT111762 | 1-0.5 | 2-1 | 2-1 | | 32-16 | 0.5 | 0.5 |
| *K. pneumoniae* | DSM30104 | 0.25 | 2-1 | 4-2 | 4 | 0.06 | 0.06-0.03 | 0.125-0.06 |
| | ATCC 700603 | 0.25 | 2-1 | 4-2 | | >64 | >0.5 | 64 |
| *S. enteritidis* | ATCC 13076 | 0.125-0.06 | 0.5 | 0.5-0.25 | 8 | 1 | 0.008 | 0.25-0.125 |
| *E.* cloacae | RKI 146/09 | 0.5-0.25 | 1 | 2 | | >64 | 0.015 | 2 |
| *S*. *marcescens* | RKI 184_11 | 2 | 4 | 4-2 | | 16 | >0.5 | 4 |
| *A. hydrophila* | ATCC7966 | 16-8 | 16 | 16 | | 0.25-0.125 | 0.001 | 0.25 |
| *M. catarrhalis* | ATCC 25238 | >64 | >64 | >64 | | 8 | 0.25-0.125 | 0.25 |
| *P. mirabilis* | ATCC 137050 | 64 | 64 | 64 | | 0.125 | 0.015 | 1 |
| *A. baumannii* | ATCC 19606 | 64 | 32 | 8 | >64 | 16 | >0.5 | 32 |

| | | | | | | | | |
|---|---|---|---|---|---|---|---|---|
| *Darobactin D22 | | | | | | | | |

Darobactin R exhibits a broad-spectrum activity against Gram-negative bacteria *in vitro* (Table 1), including key TPP pathogens *E*. *coli, K. pneumoniae* and *P. aeruginosa.* Further susceptible strains include *S*. *enteritidis, E.* cloacae and *S*. *marcescens.* Darobactin R is 8-16 fold more active against tested *E. coli* strains compared to other derivatives (B and B9 / D22). For *P. aeruginosa* the potency of darobactin R is elevated by the factor of 2-4 and for *K. pneumoniae* by the factor of 4-8 compared to B and B9. Darobactin R is highly active against MDR strains (Table 2) including colistin resistant *E. coli* NRZ14408 (*mcr-1*) or cefiderocol resistant *P. aeruginosa* EXT111762. Darobactin R is active against *E. coli* and *K. pneumoniae* in medium supplemented with 50% human serum

(Table 3). The MIC determination was done according to the method detailed under "In vitro microbiology assays", but pathogens were screened in growth medium supplemented with human serum instead of regular medium (cation-adjusted Mueller Hinton II broth).

**Table 2: MICs of darobactin R and reference compounds against MDR Gram-negative bacteria. Resistant determinants determined by whole genome sequencing. Values given in µg/mL. Pathogens were screened in growth medium supplemented with human serum instead of regular medium (cation adjusted Mueller Hinton II broth).**

| | | | MIC µg/mL | | | | | | |
|---|---|---|---|---|---|---|---|---|---|
| Strain | | Resistance determinants | DAR R | COL | AMP | CTX | MPM | GEN | FDC |
| *E.coli* | NRZ14408 | aac(6")lb-cr,aadA1,aadA2,aph(3')-Ic,ARR-3,blaKPC-2,blaOXA-1,blaTEM-1B,catA1, catB3,cmlA1,dfrA18,mcr-1,mph(A),QnrB2, strA,strB,sul1,sul3,tet(A) | 0.06 | 4 | >64 | >64 | 32 | >64 | nd |
| *E.coli* | K0416 | aac(6')lb-cr,aacA4,aadA1,aadA5,aph(3')-Ic,blaTEM-1B,blaVIM-1,catA1,dfrA17, QnrB2,strA,strB, sul1,sul2,tet(A),tet(B) | 0.03-0.06 | 0.03 | >64 | >64 | 16 | 4-2 | nd |
| *E.coli* | Survcare052 | mph(A),sul1,sul2,dfrA12,blaCTX-M-15, blaNDM-5,aadA2,strA,strB | 0.25-0.125 | 0.25 | >64 | >64 | >64 | 0.5 | nd |
| *P.aeruginosa* | EXT111762 | fosA, blaOXA-904, blaPDC-3, catB7, aph(3')-lb,gyrA, arnA | 1-0.5 | 0.25 | >64 | >64 | 64 | 1 | 16-8 |

**Table 3: MICs of darobactin R and reference compound darobactin B against reference strains screened in medium supplemented with 50% human human serum. Values are given in µg/mL.**

| **Species** | **Strain** | **DAR R** | **DAR B** |
|---|---|---|---|
| *E. coli +* 50% Serum | ATCC25922 | 0.125 | 2 |
| *K. pneumoniae +* 50% Serum | ATCC13883 | 0.25 | 4 |

### MIC₉₀ measurements:

The activity of darobactin R was determined against a cohort of *E. coli* (n=111), *K. pneumoniae* (n=143) and *P. aeruginosa* (n=121) isolates. The distribution of minimum inhibitory concentrations against all isolates is shown in Figure 1 and respective MIC₉₀ values summarized in Table 4. The activity of darobactin R is 4-fold stronger against all three species compared to reference darobactin B.

**Table 4: MIC₉₀ values of darobactin R and reference compound darobactin B against against E. coli, K. pneumoniae and P. aeruginosa.**

| | **MIC90 µg/mL** | |
|---|---|---|
| | **DAR B** | **DAR R** |
| *E.coli* (n=111) | 1 | 0.25 |
| *K. pneumoniae* (n=143) | 2 | 0.5 |
| *P. aeruginosa* (*n*=121) | 16-8 | 4 |

### Frequency of resistance:

The frequency of resistance (FoR) of *P. aeruginosa* PA01 and *E. coli* ATCC25922 in presence of 4 × MIC of darobactin R and B was determined. Results are summarized in Table 5. Gene sequencing is ongoing.

**Table 5: FoR of darobactin R and reference compound darobactin B against E. coli and P. aeruginosa.**

| | **DAR B** | **DAR R** |
|---|---|---|
| E.coli ATCC25922 | 3,92E-10 | 4,76E-09 |
| *P.aeruginosa* PAO01 | 3,60E-08 | 8,13E-07 |

### Example 3: In vitro toxicity studies

HepG2 cells were purchased from the ATCC and cultivated in DMEM High Glucose supplemented with 10% FBS. For the experiments 1% Penicillin-Streptomycin was added to the medium. Cell were seeded at 83.000 cells/mL in black/clear bottom 384 well plates. After 1 day of incubation, darobactin R was dissolved in medium and supplemented to the cells. For darobactin R, a 1:2 dilution series (9 points ranging from 100-0.5 µM) was tested. The assay plates were incubated for 72 hours, before the cell viability of determined. As a proxy for viability the metabolic capacity to reduce supplemented resazurin to resorufin (highly fluorescent). Non- viable cells cannot reduce the indicator and the respective test well does not emit a fluorescent signal. Resorufin fluorescence was measured at 535_{Ex}/590_{EM} (bottom reading) using spectrophotometer. Wells with only medium and Resazurin dye were used as background and subtracted from all other wells for the calculations. No precipitation or cell toxicity was observed at the tested concentrations. Results expressed relative to negative (maximal cell viability) control; see Figure 2.

### Example 4: ADME studies

### Metabolic stability studies:

Metabolic stability was determined in 0.5 mg/ml human (pooled of 50, mixed gener) or mouse (CD-1 males) liver microsomes at a compound concentration of 1 µM in 100 mM KPO4 buffer pH 7.4 in a total incubation volume of 500 µL. The reaction was initiated by addition of 1 mM NADPH. At various incubation times, i.e. at 0, 5, 10, 20, 40 and 60min, a sample was withdrawn from the incubation and the reaction was terminated by addition of organic solvent. The amount of parent compound remaining was determined by LC-MS/MS; see Figures 3A and 3B.

The intrinsic clearance rate (CLᵢₙₜ) for darobactin R was determined to be 4.6 µL/min/mg for HLM and MLM Half-life (t_{1/2}) of darobactin R in presence of HLM and MLM (t_{1/2}) is >300 min.

### Hemolysis studies:

Hemolytic activity of darobactin R was investigated by measuring the amount of free hemoglobin (usually contained in erythrocytes). Darobactin R was incubated in heparinized blood in triplicate. After incubation red blood cells (RBC) are removed by centrifugation. The hemoglobin concentration remaining in plasma was measured at 540 nm using a spectrophotometer. Triton X buffer 2% was used as positive control (complete lysis) and Tyrode buffer as negative control (no lysis).

At the maximum concentration of 100 µM darobactin R, no hemolysis was observed (measured 0.05 ± 0.00 %).

### Example 5: MIC data for DAR R1

The minimum inhibitory concentration of the purified darobactin derivatives, including DAR R and DAR R1 as well as reference darobactins (DAR B, DAR B9 and DAR A) were determined by microbroth dilution assays; see Table 6. Overnight cultures of the test strains were cultivated at 37°C and 180 rpm in cation adjusted Mueller Hinton II broth. Before the bacterial cultures were distributed to 96-well assay plates, the cell densities of the respective cultures were adjusted to 5 × 10⁵ cells/mL each. All compounds were dissolved in water and tested in a triplicated 12-point dilution series. In addition, dilution series of ceftazidime (CAZ), ciprofloxacin (CIP) and gentamicin (GEN) were used as positive control for each strain on each assay plate. Bacterial suspension without test compound or positive control was used as negative control e.g. growth control. Medium background was averaged from 5 replicates. Assay plates were incubated for 18h at 180 rpm and 85% relative humidity before the turbidity of all wells was determined with a microplate spectrophotometer at 600 nm (LUMIstar^{®} Omega BMG Labtech). Growth inhibition was calculated relative to the absorption of the controls. Note: In Table 6, "n=3" refers to the fact, that the individual dilution series of triplicated against each strain.

**Table 6: MICs of various darobactin derivatives against E. coli, K. pneumoniae, P. aeruginosa and A. baumannii.**

| | **MIC (µg/mL)** | | | |
|---|---|---|---|---|
| | ***Ec*** | ***Kp*** | ***Pa*** | ***Ab*** |
| n=3 | **ATCC25922** | **DSM30104** | **ATCC27853** | **ATCC19606** |
| max 64µg/mL | **MHII** | **MHII** | **MHII** | **MHII** |
| DAR A | n.d | 4 | >64 | >64 |
| DAR B | 1-0.5 | 2-1 | 8 | 32 |
| DAR B9 | 1 | 2-1 | 8 | 8 |
| DAR R | 0.06 | 0.25 | 4 | 64 |
| DAR R1 | 0.5 | 1 | 8 | >64 |

### Example 6: Structural elucidation of darobactin R

The structure of darobactin R was established by thorough 1- and 2-dimentional NMR analysis (for key correlations as well as most important peaks see Figure 4). Furthermore, comparison with the data generated for darobactin B illustrates the expected disappearance of the threonine signals, while at the same time new signals originating from the arginine residue (positions 27-32) are observed.

### NMR spectroscopy

NMR spectra (¹H, ¹³C, COSY, TOCSY, NOESY, ROESY, HSQC, HSQC-TOCSY, and HMBC) were recorded at 298 K on an Avance Neo 700 MHz spectrometer (¹H: 700.28 MHz, ¹³C: 176.09 MHz; Bruker BioSpin GmbH, Rheinstetten, Germany) equipped with a 5 mm CryoProbe Prodigy TCI (¹H, ¹⁵N, ¹³C Z-GRD). All measurements were carried out using D₂O as solvent. Chemical shifts are given in ppm. ¹H spectra were referenced to the residual solvent signal (δ = 4.79 ppm). For ¹³C measurements 3-(trimethylsilyl)propionic-2,2,3,3-d₄ acid sodium salt (TSPA, δ = 1.7 ppm) was used as external standard. For a better resolution of the correlation signals, HSQC, HSQC-TOCSY, and HMBC spectra were additionally acquired using non-uniform sampling (NUS). For TOCSY, NOESY, and ROESY spectra, experiments were carried out using H₂O suppression. Analysis of NMR spectra was achieved using the software TopSpin 3.6.0 (Bruker BioSpin GmbH, Rheinstetten, Germany).

## Claims

**1.** A compound of formula (I): 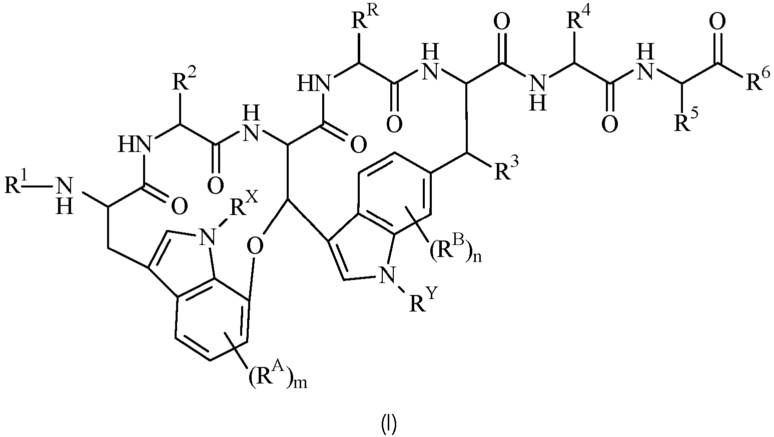 or a pharmaceutically acceptable salt thereof, wherein:
R¹ is selected from hydrogen, C₁₋₅ alkyl, C₂₋₅ alkenyl, C₂₋₅ alkynyl, C₁₋₅ haloalkyl, -(C₀₋₅ alkylene)-CO-(C₁₋₅ alkyl), -(C₀₋₅ alkylene)-CO-carbocyclyl, -(C₀₋₅ alkylene)-CO-heterocyclyl, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-CO-carbocyclyl, in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-CO-heterocyclyl, in said -(C₀₋₅ alkylene)-SO₂-heterocyclyl or in said -(C₀₋₅ alkylene)-SO₂-NH-heterocyclyl are each optionally substituted with one or more groups R^{S};
R² is selected from hydrogen, C₁₋₅ alkyl, C₂₋₅ alkenyl, C₂₋₅ alkynyl, -(C₁₋₅ alkylene)-OH, -(C₁₋₅ alkylene)-SH, -(C₁₋₅ alkylene)-S(C₁₋₅ alkyl), -(C₁₋₅ alkylene)-NH₂, -(C₁₋₅ alkylene)-NH-C(=NH)-NH₂, -(C₁₋₅ alkylene)-COOH, -(C₁₋₅ alkylene)-CO-NH₂, phenyl, -(C₁₋₅ alkylene)-phenyl, cycloalkyl, -(C₁₋₅ alkylene)-cycloalkyl, heteroaryl, -(C₁₋₅ alkylene)-heteroaryl, heterocycloalkyl, and -(C₁₋₅ alkylene)-heterocycloalkyl,
wherein said phenyl, the phenyl moiety in said -(C₁₋₅ alkylene)-phenyl, said cycloalkyl, the cycloalkyl moiety in said -(C₁₋₅ alkylene)-cycloalkyl, said heteroaryl, the heteroaryl moiety in said -(C₁₋₅ alkylene)-heteroaryl,
said heterocycloalkyl, and the heterocycloalkyl moiety in said -(C₁₋₅ alkylene)-heterocycloalkyl, are each optionally substituted with one or more groups R^{S};
R³ is selected from hydrogen, -(C₁₋₅ alkylene)-NH-R^{N}, and -(C₁₋₅ alkylene)-NH-C(=NH)-NH₂, wherein R^{N} is selected from hydrogen, C₁₋₅ alkyl, C₂₋₅ alkenyl, C₂₋₅ alkynyl, C₁₋₅ haloalkyl, -(C₀₋₅ alkylene)-CO-(C₁₋₅ alkyl), -(C₀₋₅ alkylene)-CO-carbocyclyl, -(C₀₋₅ alkylene)-CO-heterocyclyl, -(C₀₋₅ alkylene)-SO₂-(C₁₋₅ alkyl), -(C₀₋₅ alkylene)-SO₂-carbocyclyl, -(C₀₋₅ alkylene)-SO₂-heterocyclyl, -(C₀₋₅ alkylene)-SO₂-NH₂, -(C₀₋₅ alkylene)-SO₂-NH-(C₁₋₅ alkyl), -(C₀₋₅ alkylene)-SO₂-NH-carbocyclyl, and -(C₀₋₅ alkylene)-SO₂-NH-heterocyclyl, wherein the carbocyclyl moiety in said -(C₀₋₅ alkylene)-CO-carbocyclyl, in said -(C₀₋₅ alkylene)-SO₂-carbocyclyl or in said -(C₀₋₅ alkylene)-SO₂-NH-carbocyclyl and the heterocyclyl moiety in said -(C₀₋₅ alkylene)-CO-heterocyclyl, in said -(C₀₋₅ alkylene)-SO₂-heterocyclyl or in said -(C₀₋₅ alkylene)-SO₂-NH-heterocyclyl, are each optionally substituted with one or more groups R^{S};
R⁴ is selected from hydrogen, -C₁₋₅ alkyl, -(C₁₋₅ alkylene)-NH-C(=NH)-NH₂, -(C₁₋₅ alkylene)-CO-NH₂, -(C₁₋₅ alkylene)-(OH), and -(C₁₋₅ alkylene)-heterocyclyl, wherein the heterocyclyl moiety in said -(C₁₋₅ alkylene)-heterocyclyl is optionally substituted with one or more groups R^{S};
R⁵ is selected from hydrogen, C₁₋₅ alkyl, C₂₋₅ alkenyl, C₂₋₅ alkynyl, -(C₁₋₅ alkylene)-OH, -(C₁₋₅ alkylene)-SH, phenyl, -(C₁₋₅ alkylene)-phenyl, cycloalkyl, -(C₁₋₅ alkylene)-cycloalkyl, heteroaryl, -(C₁₋₅ alkylene)-heteroaryl, heterocycloalkyl, and -(C₁₋₅ alkylene)-heterocycloalkyl, wherein said phenyl, the phenyl moiety in said -(C₁₋₅ alkylene)-phenyl, said cycloalkyl, the cycloalkyl moiety in said -(C₁₋₅ alkylene)-cycloalkyl, said heteroaryl, the heteroaryl moiety in said -(C₁₋₅ alkylene)-heteroaryl, said heterocycloalkyl, and the heterocycloalkyl moiety in said -(C₁₋₅ alkylene)-heterocycloalkyl, are each optionally substituted with one or more groups R^{S};
R⁶ is selected from -OH, -NH₂, -O(C₁₋₅ alkyl), -NH(C₁₋₅ alkyl) and N(C₁₋₅ alkyl)(C₁₋₅ alkyl);
R^{R} is -(C₁₋₅ alkylene)-NH-C(=NH)-NH₂;
R^{A} and R^{B} are each individually selected from halogen, and C₁₋₅ alkyl;
R^{X} and R^{Y} are each individually selected from hydrogen, C₁₋₅ alkyl, C₁₋₅ haloalkyl, and cycloalkyl;
each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NO₂, -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -(C₀₋₃ alkylene)-carbocyclyl, and -(C₀₋₃ alkylene)-heterocyclyl, wherein the carbocyclyl moiety in said -(C₀₋₃ alkylene)-carbocyclyl and the heterocyclyl moiety in said -(C₀₋₃ alkylene)-heterocyclyl are each optionally substituted with one or more groups independently selected from C₁₋₄ alkyl, halogen, -CN, -NO₂, -OH, -O-(C₁₋₄ alkyl), -SH, -S-(C₁₋₄ alkyl), -NH₂, -NH(C₁₋₄ alkyl), -N(C₁₋₄ alkyl)(C₁₋₄ alkyl), -COOH, -COO(C₁₋₄ alkyl), -CONH₂, -CONH(C₁₋₄ alkyl), -CON(C₁₋₄ alkyl)(C₁₋₄ alkyl), -NHCO(C₁₋₄ alkyl) and -N(C₁₋₄ alkyl)-CO(C₁₋₄ alkyl);
m is 0 or 1; and
n is 0 or 1.

**2.** The compound of claim 1, which is a compound of formula (II): 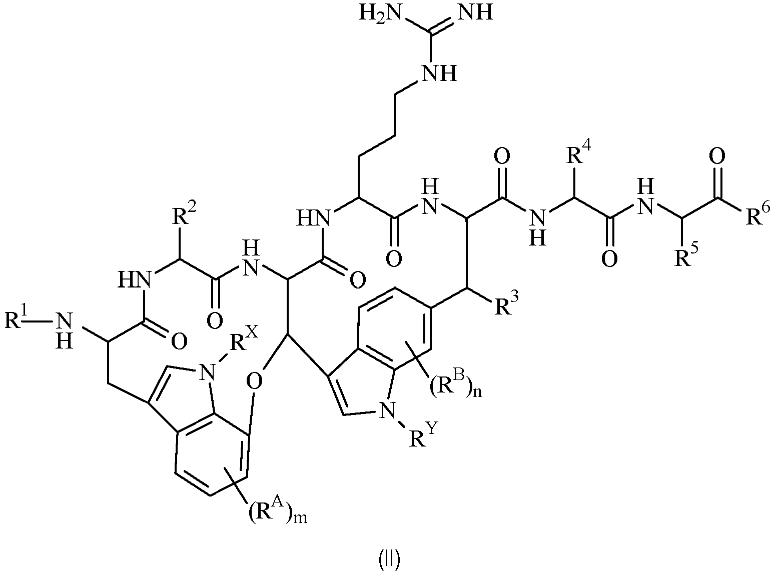 or a pharmaceutically acceptable salt thereof, wherein R¹, R², R³, R⁴, R⁵, R⁶, R^{X}, R^{Y}, R^{A}, R^{B}, m, and n are as defined in claim 1.

**3.** The compound of claim 1 or 2, wherein R¹ is H, -SO₂-methyl, or 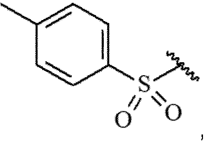 preferably wherein R¹ is H.

**4.** The compound of any one of claims 1 to 3, wherein R² is -CH₂-CO-NH₂, -CH₂CH₂-CO-NH₂, -CH(CH₃)(OH), or -CH(CH₃)(CH₂CH₃).

**5.** The compound of any one of claims 1 to 4, wherein R² is -CH₂-CO-NH₂.

**6.** The compound of any one of claims 1 to 5, wherein R³ is -CH₂CH₂CH₂-NH₂, -CH₂CH₂CH₂-NH-SO₂-CH₃, or 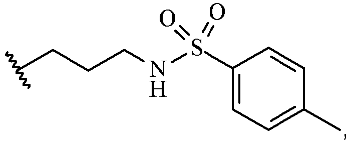 preferably wherein R³ is -CH₂CH₂CH₂-NH₂.

**7.** The compound of any one of claims 1 to 6, wherein R⁴ is selected from -CH₂CH₂CH₂-NH-C(=NH)-NH₂ and -CH(CH₃)(OH), preferably wherein R⁴ is -CH₂CH₂CH₂-NH-C(=NH)-NH₂.

**8.** The compound of any one of claims 1 to 7, wherein R⁵ is -CH(CH₃)(OH), -CH(CH₃)(CH₂CH₃), 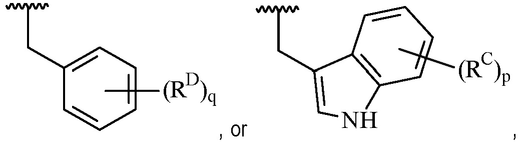 wherein R^{C} and R^{D} are each independently selected from halogen, -C₁₋₅ alkyl, and -O-C₁₋₅ alkyl, wherein p is 0 or 1 and wherein q is an integer from 0 to 5, preferably wherein R⁵ is 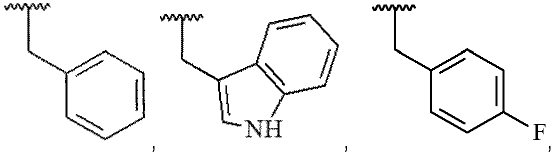 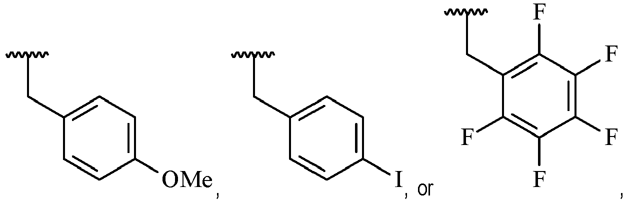 more preferably wherein R⁵ is

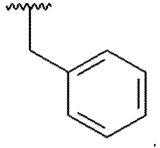

**10.** The compound of any one of claims 1 to 9,
wherein R^{X} and R^{Y} are both hydrogen, and/or
wherein m is 0 and n is 0.

**11.** The compound of claim 1 or claim 2, which is a compound selected from the following compounds: 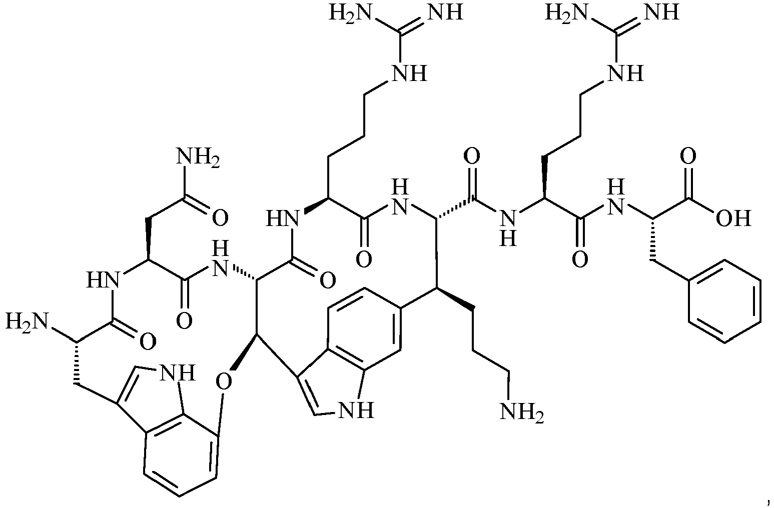 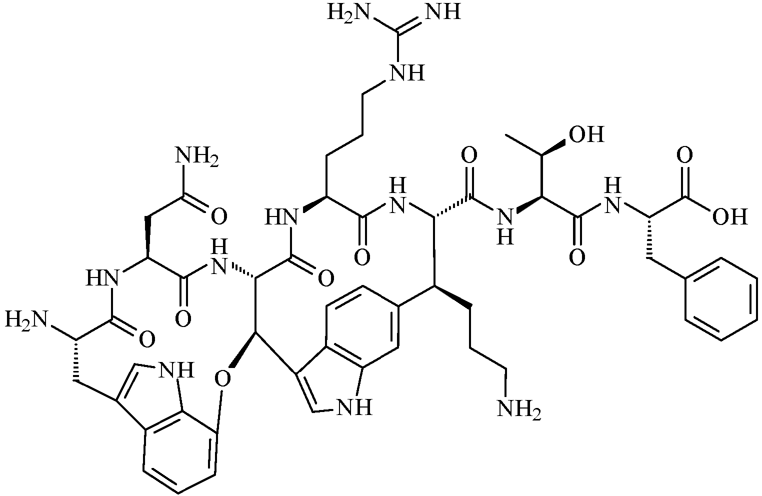 or its pharmaceutically acceptable salt.

**12.** A pharmaceutical composition comprising the compound of any one of claims 1 to 11 and a pharmaceutically acceptable carrier.

**13.** The compound of any one of claims 1 to 11 or the pharmaceutical composition of claim 12 for use as a medicament.

**14.** The compound of any one of claims 1 to 11 or the pharmaceutical composition of claim 12 for use in the treatment or prevention of a bacterial infectious disease, preferably caused by a Gram-negative bacterium.

**15.** The compound for use according to claim 14 or the pharmaceutical composition for use according to claim 14,
wherein the bacterial infectious disease is caused by one or more bacteria selected from Pseudomonas aeruginosa, Klebsiella pneumoniae, Acinetobacter baumannii, Neisseria gonorrhoeae, Chlamydia trachomatis, Shigella sonnei, Salmonella enterica Typhimurium LT2, Enterobacter cloacae, Bifidobacterium longum, Bacteroides fragilis, Lactobacillus reuteri, Enterococcus faecalis, Yersinia pestis, Pseudomonas, fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Enterobacter aerogenes, Enterobacter spp., Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter calcoaceticus, Acinetobacter haemolyticus, Yersinia enterocolitica, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria meningitidis, Kingella, Moraxella, Gardnerella vaginalis, Bacteroides distasonis, Bacteroides 3452A homology group, Bacteroides vulgatus, Bacteroides ovalus, Bacteroides thetaiotaomicron, Bacteroides uniformis, Bacteroides eggerthii, and Bacteroides splanchnicus,
and/or
wherein the compound or the pharmaceutical composition is to be administered in combination with one or more of an antibiotic, an antifungal agent, an anthelmintic agent, an antimalarial agent, an antiprotozoal agent, an antituberculosis agent, and an antiviral agent.
